# EUROPEAN PATENT APPLICATION

(11) **EP 1 733 743 A1**
(43) Date of publication of application: **20.12.2006**
(21) Application number: 05730699.5
(22) Date of filing: 06.04.2005
(51) Int. Cl.: A61K 48/00, A61K 31/7088, A61K 38/00, A61K 39/395, A61K 45/00, A61P 35/00, A61P 43/00, G01N 33/15, G01N 33/50, G01N 33/53

(54) **PREVENTIVES/REMEDIES FOR CANCER**

(30) Priority: 09.04.2004 JP 2004114855
(71) Applicant: Takeda Pharmaceutical Company Limited, Osaka-shi, Osaka 5408645 (JP)
(72) Inventor: SATOU, Shuuji, TAKEDA PHARMACEUTICAL COMPANY LTD., Osaka-shi, Osaka 5328686 (JP); ISHII, Takafumi, TAKEDA PHARMACEUTICAL COMP. LTD., Osaka-shi, Osaka 5328686 (JP)
(74) Representative: Lewin, John Harvey
(86) International application number: PCT/JP2005/007112
(87) International publication number: WO 2005/097204

(57) **Abstract**

The present invention provides preventives/remedies for cancer and so on. Specifically, an antibody against a protein comprising the same or substantially the same as the amino acid sequence represented by SEQ ID NO: 1, SEQ ID NO: 3, SEQ ID NO: 17, SEQ ID NO: 25, SEQ ID NO: 38 or SEQ ID NO: 48, a compound that inhibits the expression of the above protein or the expression of a gene for the above protein, and so on, are useful as preventives/remedies for cancer, etc., cancer cell apoptosis promoters, cancer cell growth inhibitors, and so on.

SEQUENCE LISTING

<110> Takeda Pharmaceutical Company Limited

<120> PREVENTIVES/REMEDIES FOR CANCER

<130> G06-0043

<150> JP 2004-114855
<151> 2004-04-09

<160> 71

<210> 1
<211> 479
<212> PRT
<213> Homo sapiens

<400> 1
Met Ala Arg Ala Ala Ala Leu Leu Pro Ser Arg Ser Pro Pro Thr Pro
                5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His
        115                 120                 125
Gly Leu Thr Val Glu Asp Glu Gly Asn Tyr Thr Cys Glu Phe Ala Thr
    130                 135                 140

Phe Pro Lys Gly Ser Val Arg Gly Met Thr Trp Leu Arg Val Ile Ala
145                 150                 155                 160
Lys Pro Lys Asn Gln Ala Glu Ala Gln Lys Val Thr Phe Ser Gln Asp
                165                 170                 175
Pro Thr Thr Val Ala Leu Cys Ile Ser Lys Glu Gly Arg Pro Pro Ala
            180                 185                 190
Arg Ile Ser Trp Leu Ser Ser Leu Asp Trp Glu Ala Lys Glu Thr Gln
        195                 200                 205
Val Ser Gly Thr Leu Ala Gly Thr Val Thr Val Thr Ser Arg Phe Thr
    210                 215                 220
Leu Val Pro Ser Gly Arg Ala Asp Gly Val Thr Val Thr Cys Lys Val
225                 230                 235                 240
Glu His Glu Ser Phe Glu Glu Pro Ala Leu Ile Pro Val Thr Leu Ser
                245                 250                 255
Val Arg Tyr Pro Pro Glu Val Ser Ile Ser Gly Tyr Asp Asp Asn Trp
            260                 265                 270
Tyr Leu Gly Arg Thr Asp Ala Thr Leu Ser Cys Asp Val Arg Ser Asn
        275                 280                 285
Pro Glu Pro Thr Gly Tyr Asp Trp Ser Thr Thr Ser Gly Thr Phe Pro
    290                 295                 300
Thr Ser Ala Val Ala Gln Gly Ser Gln Leu Val Ile His Ala Val Asp
305                 310                 315                 320
Ser Leu Phe Asn Thr Thr Phe Val Cys Thr Val Thr Asn Ala Val Gly
                325                 330                 335
Met Gly Arg Ala Glu Gln Val Ile Phe Val Arg Glu Thr Pro Arg Ala
            340                 345                 350
Ser Pro Arg Asp Val Gly Pro Leu Val Trp Gly Ala Val Gly Gly Thr
        355                 360                 365
Leu Leu Val Leu Leu Leu Leu Ala Gly Gly Ser Leu Ala Phe Ile Leu
    370                 375                 380
Leu Arg Val Arg Arg Arg Arg Lys Ser Pro Gly Gly Ala Gly Gly Gly
385                 390                 395                 400
Ala Ser Gly Asp Gly Gly Phe Tyr Asp Pro Lys Ala Gln Val Leu Gly
                405                 410                 415
Asn Gly Asp Pro Val Phe Trp Thr Pro Val Val Pro Gly Pro Met Glu
            420                 425                 430
Pro Asp Gly Lys Asp Glu Glu Glu Glu Glu Glu Glu Glu Lys Ala Glu

435                 440                 445
Lys Gly Leu Met Leu Pro Pro Pro Pro Ala Leu Glu Asp Asp Met Glu
    450                 455                 460
Ser Gln Leu Asp Gly Ser Leu Ile Ser Arg Arg Ala Val Tyr Val
465                 470                 475

<210> 2
<211> 1437
<212> DNA
<213> Homo sapiens

<400> 2
atggcccggg ccgctgccct cctgccgtcg agatcgccgc cgacgccgct gctgtggccg      60
ctgctgctgc tgctgctcct ggaaaccgga gcccaggatg tgcgagttca agtgctaccc     120
gaggtgcgag gccagctcgg gggcaccgtg gagctgccgt gccacctgct gccacctgtt     180
cctggactgt acatctccct ggtgacctgg cagcgcccag atgcacctgc gaaccaccag     240
aatgtggccg ccttccaccc taagatgggt cccagcttcc ccagcccgaa gcctggcagc     300
gagcggctgt ccttcgtctc tgccaagcag agcactgggc aagacacaga ggcagagctc     360
caggacgcca cgctggccct ccacgggctc acggtggagg acgagggcaa ctacacttgc     420
gagtttgcca ccttccccaa ggggtccgtc cgagggatga cctggctcag agtcatagcc     480
aagcccaaga accaagctga ggcccagaag gtcacgttca gccaggaccc tacgacagtg     540
gccctctgca tctccaaaga gggccgccca cctgcccgga tctcctggct ctcatccctg     600
gactgggaag ccaaagagac tcaggtgtca gggaccctgg ccggaactgt cactgtcacc     660
agccgcttca ccttggtgcc ctcgggccga gcagatggtg tcacggtcac ctgcaaagtg     720
gagcatgaga gcttcgagga accagccctg atacctgtga ccctctctgt acgctaccct     780
cctgaagtgt ccatctccgg ctatgatgac aactggtacc tcggccgtac tgatgccacc     840
ctgagctgtg acgtccgcag caacccagag cccacgggct atgactggag cacgacctca     900
ggcaccttcc cgacctccgc agtggcccag ggctcccagc tggtcatcca cgcagtggac     960
agtctgttca ataccacctt cgtctgcaca gtcaccaatg ccgtgggcat gggccgcgct    1020
gagcaggtca tctttgtccg agaaaccccc agggcctcgc cccgagatgt gggcccgctg    1080
gtgtgggggg ccgtgggggg gacactgctg gtgctgctgc ttctggctgg ggggtccttg    1140
gccttcatcc tgctgagggt gaggaggagg aggaagagcc ctggaggagc aggaggagga    1200
gccagtggcg acgggggatt ctacgatccg aaagctcagg tgttgggaaa tggggacccc    1260
gtcttctgga caccagtagt ccctggtccc atggaaccag atggcaagga tgaggaggag    1320
gaggaggagg aagagaaggc agagaaaggc ctcatgttgc ctccaccccc agcactcgag    1380
gatgacatgg agtcccagct ggacggctcc ctcatctcac ggcgggcagt ttatgtg       1437

<210> 3
<211> 538
<212> PRT
<213> Homo sapiens

<400> 3
Met Ala Arg Ala Ala Ala Leu Leu Pro Ser Arg Ser Pro Pro Thr Pro
                5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His
        115                 120                 125
Gly Leu Thr Val Glu Asp Glu Gly Asn Tyr Thr Cys Glu Phe Ala Thr
    130                 135                 140
Phe Pro Lys Gly Ser Val Arg Gly Met Thr Trp Leu Arg Val Ile Ala
145                 150                 155                 160
Lys Pro Lys Asn Gln Ala Glu Ala Gln Lys Val Thr Phe Ser Gln Asp
                165                 170                 175
Pro Thr Thr Val Ala Leu Cys Ile Ser Lys Glu Gly Arg Pro Pro Ala
            180                 185                 190
Arg Ile Ser Trp Leu Ser Ser Leu Asp Trp Glu Ala Lys Glu Thr Gln
        195                 200                 205
Val Ser Gly Thr Leu Ala Gly Thr Val Thr Val Thr Ser Arg Phe Thr
    210                 215                 220
Leu Val Pro Ser Gly Arg Ala Asp Gly Val Thr Val Thr Cys Lys Val
225                 230                 235                 240
Glu His Glu Ser Phe Glu Glu Pro Ala Leu Ile Pro Val Thr Leu Ser

                245                 250                 255
Val Arg Tyr Pro Pro Glu Val Ser Ile Ser Gly Tyr Asp Asp Asn Trp
            260                 265                 270
Tyr Leu Gly Arg Thr Asp Ala Thr Leu Ser Cys Asp Val Arg Ser Asn
        275                 280                 285
Pro Glu Pro Thr Gly Tyr Asp Trp Ser Thr Thr Ser Gly Thr Phe Pro
    290                 295                 300
Thr Ser Ala Val Ala Gln Gly Ser Gln Leu Val Ile His Ala Val Asp
305                 310                 315                 320
Ser Leu Phe Asn Thr Thr Phe Val Cys Thr Val Thr Asn Ala Val Gly
                325                 330                 335
Met Gly Arg Ala Glu Gln Val Ile Phe Val Arg Glu Thr Pro Asn Thr
            340                 345                 350
Ala Gly Ala Gly Ala Thr Gly Gly Ile Ile Gly Gly Ile Ile Ala Ala
        355                 360                 365
Ile Ile Ala Thr Ala Val Ala Ala Thr Gly Ile Leu Ile Cys Arg Gln
    370                 375                 380
Gln Arg Lys Glu Gln Thr Leu Gln Gly Ala Glu Glu Asp Glu Asp Leu
385                 390                 395                 400
Glu Gly Pro Pro Ser Tyr Lys Pro Pro Thr Pro Lys Ala Lys Leu Glu
                405                 410                 415
Ala Gln Glu Met Pro Ser Gln Leu Phe Thr Leu Gly Ala Ser Glu His
            420                 425                 430
Ser Pro Leu Lys Thr Pro Tyr Phe Asp Ala Gly Ala Ser Cys Thr Glu
        435                 440                 445
Gln Glu Met Pro Arg Tyr His Glu Leu Pro Thr Leu Glu Glu Arg Ser
    450                 455                 460
Gly Pro Leu His Pro Gly Ala Thr Ser Leu Gly Ser Pro Ile Pro Val
465                 470                 475                 480
Pro Pro Gly Pro Pro Ala Val Glu Asp Val Ser Leu Asp Leu Glu Asp
                485                 490                 495
Glu Glu Gly Glu Glu Glu Glu Glu Tyr Leu Asp Lys Ile Asn Pro Ile
            500                 505                 510
Tyr Asp Ala Leu Ser Tyr Ser Ser Pro Ser Asp Ser Tyr Gln Gly Lys
        515                 520                 525
Gly Phe Val Met Ser Arg Ala Met Tyr Val
    530                 535

<210> 4
<211> 1614
<212> DNA
<213> Homo sapiens

<400> 4
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatttccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agagaccccc aacacagcag gcgcaggggc cacaggcggc 1080
atcatcgggg gcatcatcgc cgccatcatt gctactgctg tggctgccac gggcatcctt 1140
atctgccggc agcagcggaa ggagcagacg ctgcaggggg cagaggagga cgaagacctg 1200
gagggacctc cctcctacaa gccaccgacc ccaaaagcga agctggaggc acaggagatg 1260
ccctcccagc tcttcactct gggggcctcg gagcacagcc cactcaagac cccctacttt 1320
gatgctggcg cctcatgcac tgagcaggaa atgcctcgat accatgagct gcccaccttg 1380
gaagaacggt caggaccctt gcaccctgga gccacaagcc tggggtcccc catcccggtg 1440
cctccagggc cacctgctgt ggaagacgtt tccctggatc tagaggatga ggagggggag 1500
gaggaggaag agtatctgga caagatcaac cccatctatg atgctctgtc ctatagcagc 1560
ccctctgatt cctaccaggg caaaggcttt gtcatgtccc gggccatgta tgtg 1614

<210> 5
<211> 20

<212> DNA
<213> Artificial Sequence

<220>
<223> Antisense oligonucleotide 1

<400> 5
tcccaacacc tgagctttcg                                                    20

<210> 6
<211> 20
<212> DNA
<213> Artificial Sequence

<220>
<223> Control oligonucleotide 1

<400> 6
gctttcgagt ccacaaccct                                                    20

<210> 7
<211> 22
<212> DNA
<213> Artificial Sequence

<220>
<223> Primer

<400> 7
ggtcatcttt gtccgagaaa cc                                                 22

<210> 8
<211> 22
<212> DNA
<213> Artificial Sequence

<220>

<223> Primer

<400> 8
tgagctttcg gatcgtagaa tc                                                22

<210> 9
<211> 18
<212> DNA
<213> Artificial Sequence

<220>
<223> TaqMan Probe 1

<400> 9
ccgagatgtg ggcccgct                                                     18

<210> 10
<211> 22
<212> DNA
<213> Artificial Sequence

<220>
<223> Primer

<400> 10
   cccactcaag accccctact tt                                               22

<210> 11
<211> 22
<212> DNA
<213> Artificial Sequence

<220>
<223> Primer

<400> 11
gctcatggta tcgaggcatt tc                                                22

<210> 12
<211> 23
<212> DNA
<213> Artificial Sequence

<220>
<223> TaqMan Probe 2

<400> 12
atgctggcgc ctcatgcact gag                                                23

<210> 13
<211> 25
<212> DNA
<213> Artificial Sequence

<220>
<223> Primer

<400> 13
aattgaattc atggcccggg ccgct                                              25

<210> 14
<211> 32
<212> DNA
<213> Artificial Sequence

<220>
<223> Primer

<400> 14
aattgatatc tcacacataa actgcccgcc gt                                      32

<210> 15
<211> 29
<212> DNA

<213> Artificial Sequence

<220>
<223> Primer

<400> 15
gatatctcac acatacatgg cccgggaca                                        29

<210> 16
<211> 34
<212> DNA
<213> Artificial Sequence

<220>
<223> Primer

<400> 16
attgatatct cacacataca tggcccggga catg                                  34

<210> 17
<211> 344
<212> PRT
<213> Homo sapiens

<400> 17
Met Phe Thr Ser Thr Gly Ser Ser Gly Leu Tyr Lys Ala Pro Leu Ser
                5                   10                  15
Lys Ser Leu Leu Leu Val Pro Ser Ala Leu Ser Leu Leu Leu Ala Leu
            20                  25                  30
Leu Leu Pro His Cys Gln Lys Leu Phe Val Tyr Asp Leu His Ala Val
        35                  40                  45
Lys Asn Asp Phe Gln Ile Trp Arg Leu Ile Cys Gly Arg Ile Ile Cys
    50                  55                  60
Leu Asp Leu Lys Asp Thr Phe Cys Ser Ser Leu Leu Ile Tyr Asn Phe
65                  70                  75                  80
Arg Ile Phe Glu Arg Arg Tyr Gly Ser Arg Lys Phe Ala Ser Phe Leu

85                  90                  95
Leu Gly Ser Trp Val Leu Ser Ala Leu Phe Asp Phe Leu Leu Ile Glu
            100                 105                 110
Ala Met Gln Tyr Phe Phe Gly Ile Thr Ala Ala Ser Asn Leu Pro Ser
        115                 120                 125
Gly Phe Leu Ala Pro Val Phe Ala Leu Phe Val Pro Phe Tyr Cys Ser
    130                 135                 140
Ile Pro Arg Val Gln Val Ala Gln Ile Leu Gly Pro Leu Ser Ile Thr
145                 150                 155                 160
Asn Lys Thr Leu Ile Tyr Ile Leu Gly Leu Gln Leu Phe Thr Ser Gly
                165                 170                 175
Ser Tyr Ile Trp Ile Val Ala Ile Ser Gly Leu Met Ser Gly Leu Cys
            180                 185                 190
Tyr Asp Ser Lys Met Phe Gln Val His Gln Val Leu Cys Ile Pro Ser
        195                 200                 205
Trp Met Ala Lys Phe Phe Ser Trp Thr Leu Glu Pro Ile Phe Ser Ser
    210                 215                 220
Ser Glu Pro Thr Ser Glu Ala Arg Ile Gly Met Gly Ala Thr Leu Asp
225                 230                 235                 240
Ile Gln Arg Gln Gln Arg Met Glu Leu Leu Asp Arg Gln Leu Met Phe
                245                 250                 255
Ser Gln Phe Ala Gln Gly Arg Arg Gln Arg Gln Gln Gln Gly Gly Met
            260                 265                 270
Ile Asn Trp Asn Arg Leu Phe Pro Pro Leu Arg Gln Arg Gln Asn Val
        275                 280                 285
Asn Tyr Gln Gly Gly Arg Gln Ser Glu Pro Ala Ala Pro Pro Leu Glu
    290                 295                 300
Val Ser Glu Glu Gln Val Ala Arg Leu Met Glu Met Gly Phe Ser Arg
305                 310                 315                 320
Gly Asp Ala Leu Glu Ala Leu Arg Ala Ser Asn Asn Asp Leu Asn Val
                325                 330                 335
Ala Thr Asn Phe Leu Leu Gln His
            340

<210> 18
<211> 1032
<212> DNA

<213> Homo sapiens

<400> 18
atgttcacca gcaccggctc cagtgggctc tacaaggcgc ctctgtcgaa gagccttctg 60
ctggtcccca gtgccctctc cctcctgctc gccctcctcc tgcctcactg ccagaagctc 120
tttgtgtatg accttcacgc agtcaagaac gacttccaga tttggaggtt gatatgtgga 180
agaataattt gccttgattt gaaagatact ttctgcagta gtctgcttat ttataatttt 240
aggatatttg aaagaagata tggaagcaga aaatttgcat cctttttgct gggttcctgg 300
gttttgtcag ccttatttga ctttctcctc attgaagcta tgcagtattt ctttggcatc 360
actgcagcta gtaatttgcc ttctggattc ctggcacctg tgtttgctct gtttgtacca 420
ttttactgct ccataccaag agtccaagtg gcacaaattc tgggtccgtt gtccatcaca 480
aacaagacat tgatttatat attgggactg cagcttttca cctctggttc ctacatctgg 540
attgtagcca taagtggact tatgtccggt ctgtgctacg acagcaaaat gttccaggtg 600
catcaggtgc tctgcatccc cagctggatg gcaaaattct tttcttggac acttgaaccc 660
atcttctctt cttcagaacc caccagcgaa gccagaattg ggatgggagc cacgctggac 720
atccagagac agcagagaat ggagctgctg gaccggcagc tgatgttctc tcagtttgca 780
caagggaggc gacagagaca gcagcaggga ggaatgatca attggaatcg tctttttcct 840
cctttacgtc agcgacaaaa cgtaaactat cagggcggtc ggcagtctga gccagcagcg 900
ccccctctag aagtttctga ggaacaggtc gcccggctca tggagatggg attttccaga 960
ggtgatgctt tggaagccct gagagcttca aacaatgacc tcaatgtcgc caccaacttc 1020
ctgctgcagc ac 1032

<210> 19
<211> 20
<212> DNA
<213> Artificial Sequence

<220>
<223> Antisense oligonucleotide 2

<400> 19
accctctccc catcacgacg 20

<210> 20
<211> 20
<212> DNA
<213> Artificial Sequence

<220>
<223> Control oligonucleotide 2

<400> 20
gcagcactac ccctctccca                                                    20

<210> 21
<211> 25
<212> DNA
<213> Artificial Sequence

<220>
<223> Primer

<400> 21
ctgtttgtac cattttactg ctcca                                              25

<210> 22
<211> 25
<212> DNA
<213> Artificial Sequence

<220>
<223> Primer

<400> 22
gtccacttat ggctacaatc cagat                                              25

<210> 23
<211> 24
<212> DNA
<213> Artificial Sequence

<220>
<223> Primer

<400> 23
gaattcatgt tcaccagcac cggc                                              24

<210> 24
<211> 27
<212> DNA
<213> Artificial Sequence

<220>
<223> Primer

<400> 24
ctcgagtcag tgctgcagca ggaagtt                                           27

<210> 25
<211> 292
<212> PRT
<213> Homo sapiens

<400> 25
Met Leu Gly Ala Trp Ala Val Glu Gly Thr Ala Val Ala Leu Leu Arg
                5                   10                  15
Leu Leu Leu Leu Leu Leu Pro Pro Ala Ile Arg Gly Pro Gly Leu Gly
            20                  25                  30
Val Ala Gly Val Ala Gly Ala Ala Gly Ala Gly Leu Pro Glu Ser Val
        35                  40                  45
Ile Trp Ala Val Asn Ala Gly Gly Glu Ala His Val Asp Val His Gly
    50                  55                  60
Ile His Phe Arg Lys Asp Pro Leu Glu Gly Arg Val Gly Arg Ala Ser
65                  70                  75                  80
Asp Tyr Gly Met Lys Leu Pro Ile Leu Arg Ser Asn Pro Glu Asp Gln
                85                  90                  95
Ile Leu Tyr Gln Thr Glu Arg Tyr Asn Glu Glu Thr Phe Gly Tyr Glu
            100                 105                 110
Val Pro Ile Lys Glu Glu Gly Asp Tyr Val Leu Val Leu Lys Phe Ala
        115                 120                 125
Glu Val Tyr Phe Ala Gln Ser Gln Gln Lys Val Phe Asp Val Arg Leu

130                 135                 140
Asn Gly His Val Val Val Lys Asp Leu Asp Ile Phe Asp Arg Val Gly
145                 150                 155                 160
His Ser Thr Ala His Asp Glu Ile Ile Pro Met Ser Ile Arg Lys Gly
                165                 170                 175
Lys Leu Ser Val Gln Gly Glu Val Ser Thr Phe Thr Gly Lys Leu Tyr
            180                 185                 190
Ile Glu Phe Val Lys Gly Tyr Tyr Asp Asn Pro Lys Val Cys Ala Leu
        195                 200                 205
Tyr Ile Met Ala Gly Thr Val Asp Asp Val Pro Lys Leu Gln Pro His
    210                 215                 220
Pro Gly Leu Glu Lys Lys Glu Glu Glu Glu Glu Glu Glu Glu Tyr Asp
225                 230                 235                 240
Glu Gly Ser Asn Leu Lys Lys Gln Thr Asn Lys Asn Arg Val Gln Ser
                245                 250                 255
Gly Pro Arg Thr Pro Asn Pro Tyr Ala Ser Asp Asn Ser Ser Leu Met
            260                 265                 270
Phe Pro Ile Leu Val Ala Phe Gly Val Phe Ile Pro Thr Leu Phe Cys
        275                 280                 285
Leu Cys Arg Leu
    290

<210> 26
<211> 876
<212> DNA
<213> Homo sapiens

<400> 26
atgctgggag cctgggcggt tgagggaacc gctgtggcgc tcctgcgact gctgctgctg      60
ctgctgccgc cggcgatccg gggacccggg ctcggcgtgg ccggcgtggc cggcgcggcg     120
ggggccgggc tgcccgagag cgtcatttgg gcggtcaacg cgggtggaga ggcgcatgtg     180
gacgtgcacg ggatccactt ccgcaaggac cctttggaag gccgggtggg ccgagcctca     240
gactatggca tgaaactgcc aatcctgcgt tccaaccctg aggaccagat cctgtatcaa     300
actgagcggt acaatgagga gacctttggc tacgaagtgc ccatcaaaga ggagggggac     360
tacgtgctgg tcttgaaatt tgcagaggtc tactttgcac agtcccagca aaaggtattt     420
gatgtacgat tgaatggcca cgtcgtggtg aaggacttgg atatctttga tcgtgttggg     480
catagcacag ctcacgatga aattatacct atgagcatca gaaaggggaa gctgagtgtc     540

cagggggagg tgtccacctt cacagggaaa ctctacattg agtttgtcaa ggggtactat 600
gacaatccca aggtctgtgc actctacatc atggctggga cagtggatga tgtaccaaag 660
cttcagcctc atccgggatt ggagaagaaa gaagaggaag aagaagaaga agaatatgat 720
gaagggtcta atctcaaaaa acagaccaat aagaaccggg tgcagtcagg cccccgcaca 780
cccaacccct atgcctcgga caacagcagc ctcatgtttc ccatcctggt ggccttcgga 840
gtcttcattc caaccctctt ctgcctctgc cggttg 876

<210> 27
<211> 20
<212> DNA
<213> Artificial Sequence

<220>
<223> Antisense oligonucleotide 3

<400> 27
gtcccatcac accaagaacc 20

<210> 28
<211> 20
<212> DNA
<213> Artificial Sequence

<220>
<223> Control oligonucleotide 3

<400> 28
ccaagaacca cactaccctg 20

<210> 29
<211> 20
<212> DNA
<213> Artificial Sequence

<220>
<223> Primer

<400> 29
gttgggcata gcacagctca                                                    20

<210> 30
<211> 21
<212> DNA
<213> Artificial Sequence

<220>
<223> Primer

<400> 30
gcacagacct tgggattgtc a                                                  21

<210> 31
<211> 27
<212> DNA
<213> Artificial Sequence

<220>
<223> Primer

<400> 31
ctcccttctt ccagtgcctt gtgacag                                            27

<210> 32
<211> 29
<212> DNA
<213> Artificial Sequence

<220>
<223> Primer

<400> 32
aattgaattc atggtgcccg gcgcccgcg                                          29

<210> 33

<211> 28
<212> DNA
<213> Artificial Sequence

<220>
<223> Primer

<400> 33
aattctcgag tcacaaaagg gcagtcat                                          28

<210> 34
<211> 30
<212> DNA
<213> Artificial Sequence

<220>
<223> Primer

<400> 34
gaattcatgc tgggagcctg ggcggttgag                                        30

<210> 35
<211> 22
<212> DNA
<213> Artificial Sequence

<220>
<223> Primer

<400> 35
tctagatcac aaccggcaga gg                                                22

<210> 36
<211> 25
<212> DNA
<213> Artificial Sequence

<220>
<223> Primer

<400> 36
aattgaattc atgctgggag cctgg                                          25

<210> 37
<211> 26
<212> DNA
<213> Artificial Sequence

<220>
<223> Primer

<400> 37
aattctcgag tcacaaccgg cagagg                                         26

<210> 38
<211> 719
<212> PRT
<213> Homo sapiens

<400> 38
Met Ala Ala Leu Glu Glu Glu Phe Thr Leu Ser Ser Val Val Leu Ser
                5                   10                  15
Ala Gly Pro Glu Gly Leu Leu Gly Val Glu Gln Ser Asp Lys Thr Asp
            20                  25                  30
Gln Phe Leu Val Thr Asp Ser Gly Arg Thr Val Ile Leu Tyr Lys Val
        35                  40                  45
Ser Asp Gln Lys Pro Leu Gly Ser Trp Ser Val Lys Gln Gly Gln Ile
    50                  55                  60
Ile Thr Cys Pro Ala Val Cys Asn Phe Gln Thr Gly Glu Tyr Val Val
65                  70                  75                  80
Val His Asp Asn Lys Val Leu Arg Ile Trp Asn Asn Glu Asp Val Asn
                85                  90                  95
Leu Asp Lys Val Phe Lys Ala Thr Leu Ser Ala Glu Val Tyr Arg Ile
            100                 105                 110

Leu Ser Val Gln Gly Thr Glu Pro Leu Val Leu Phe Lys Glu Gly Ala
        115                 120                 125
Val Arg Gly Leu Glu Ala Leu Leu Ala Asp Pro Gln Gln Lys Ile Glu
    130                 135                 140
Thr Val Ile Ser Asp Glu Glu Val Ile Lys Trp Thr Lys Phe Phe Val
145                 150                 155                 160
Val Phe Arg His Pro Val Leu Ile Phe Ile Thr Glu Lys His Gly Asn
                165                 170                 175
Tyr Phe Ala Tyr Val Gln Met Phe Asn Ser Arg Ile Leu Thr Lys Tyr
            180                 185                 190
Thr Leu Leu Leu Gly Gln Asp Glu Asn Ser Val Ile Lys Ser Phe Thr
        195                 200                 205
Ala Ser Val Asp Arg Lys Phe Ile Ser Leu Met Ser Leu Ser Ser Asp
    210                 215                 220
Gly Cys Ile Tyr Glu Thr Leu Ile Pro Ile Arg Pro Ala Asp Pro Glu
225                 230                 235                 240
Lys Asn Gln Ser Leu Val Lys Ser Leu Leu Leu Lys Ala Val Val Ser
                245                 250                 255
Gly Asn Ala Arg Asn Gly Val Ala Leu Thr Ala Leu Asp Gln Asp His
            260                 265                 270
Val Ala Val Leu Gly Ser Pro Leu Ala Ala Ser Lys Glu Cys Leu Ser
        275                 280                 285
Val Trp Asn Ile Lys Phe Gln Thr Leu Gln Thr Ser Lys Glu Leu Pro
    290                 295                 300
Gln Gly Thr Ser Gly Gln Leu Trp Tyr Tyr Gly Glu His Leu Phe Met
305                 310                 315                 320
Leu His Gly Lys Ser Leu Thr Val Ile Pro Tyr Lys Cys Glu Val Ser
                325                 330                 335
Ser Leu Ala Gly Ala Leu Gly Lys Leu Lys His Ser Gln Asp Pro Gly
            340                 345                 350
Thr His Val Val Ser His Phe Val Asn Trp Glu Thr Pro Gln Gly Cys
        355                 360                 365
Gly Leu Gly Phe Gln Asn Ser Glu Gln Ser Arg Arg Ile Leu Arg Arg
    370                 375                 380
Arg Lys Ile Glu Val Ser Leu Gln Pro Glu Val Pro Pro Ser Lys Gln
385                 390                 395                 400
Leu Leu Ser Thr Ile Met Lys Asp Ser Glu Lys His Ile Glu Val Glu

                405                 410                 415
Val Arg Lys Phe Leu Ala Leu Lys Gln Thr Pro Asp Phe His Thr Val
            420                 425                 430
Ile Gly Asp Thr Val Thr Gly Leu Leu Glu Arg Cys Lys Ala Glu Pro
        435                 440                 445
Ser Phe Tyr Pro Arg Asn Cys Leu Met Gln Leu Ile Gln Thr His Val
    450                 455                 460
Leu Ser Tyr Ser Leu Cys Pro Asp Leu Met Glu Ile Ala Leu Lys Lys
465                 470                 475                 480
Lys Asp Val Gln Leu Leu Gln Leu Cys Leu Gln Gln Phe Pro Asp Ile
                485                 490                 495
Pro Glu Ser Val Thr Cys Ala Cys Leu Lys Ile Phe Leu Ser Ile Gly
            500                 505                 510
Asp Asp Ser Leu Gln Glu Thr Asp Val Asn Met Glu Ser Val Phe Asp
        515                 520                 525
Tyr Ser Ile Asn Ser Val His Asp Glu Lys Met Glu Glu Gln Thr Glu
    530                 535                 540
Ile Leu Gln Asn Gly Phe Asn Pro Glu Glu Asp Lys Cys Asn Asn Cys
545                 550                 555                 560
Asp Gln Glu Leu Asn Lys Lys Pro Gln Asp Glu Thr Lys Glu Ser Thr
                565                 570                 575
Ser Cys Pro Val Val Gln Lys Arg Ala Ala Leu Leu Asn Ala Ile Leu
            580                 585                 590
His Ser Ala Tyr Ser Glu Thr Phe Leu Leu Pro His Leu Lys Asp Ile
        595                 600                 605
Pro Ala Gln His Ile Thr Leu Phe Leu Lys Tyr Leu Tyr Phe Leu Tyr
    610                 615                 620
Leu Lys Cys Ser Glu Asn Ala Thr Met Thr Leu Pro Gly Ile His Pro
625                 630                 635                 640
Pro Thr Leu Asn Gln Ile Met Asp Trp Ile Cys Leu Leu Leu Asp Ala
                645                 650                 655
Asn Phe Thr Val Val Val Met Met Pro Glu Ala Lys Arg Leu Leu Ile
            660                 665                 670
Asn Leu Tyr Lys Leu Val Lys Ser Gln Ile Ser Val Tyr Ser Glu Leu
        675                 680                 685
Asn Lys Ile Glu Val Ser Phe Arg Glu Leu Gln Lys Leu Asn Gln Glu
    690                 695                 700

Lys Asn Asn Arg Gly Leu Tyr Ser Ile Glu Val Leu Glu Leu Phe
705                 710                 715

<210> 39
<211> 2157
<212> DNA
<213> Homo sapiens

<400> 39
atggcagcgc tggaggaaga attcacgttg tcttcggtag tcctgagcgc cgggcctgaa    60
ggactcctag gcgtggagca gagcgacaaa acagaccagt ttctagtgac agacagcggc   120
aggacagtca tcctctataa ggtttctgat cagaaaccct tggggagctg gtcagtgaaa   180
caaggtcaaa ttataacatg tccagctgtg tgcaactttc aaactggaga gtatgttgtt   240
gtacacgata ataaggtttt aagaatatgg aataatgaag atgtaaacct ggataaagta   300
tttaaagcta cattgtcagc agaagtatat aggatacttt cagtgcaagg gacagaaccc   360
ttggtgctct tcaaggaagg tgctgttcgt ggtttagagg ccttgcttgc agacccccag   420
cagaaaattg aaactgttat ctctgatgaa gaagtgatta aatggacaaa gtttttcgta   480
gtattcagac atcctgtttt aatttttatt actgaaaaac atggaaatta ctttgcttac   540
gtgcaaatgt ttaactcacg tatcttaacc aaatatacac tcttacttgg acaagacgaa   600
aactctgtta taaagagttt tactgcatct gtagatcgga aattcatctc tttgatgtca   660
ttaagctctg atggttgtat atatgaaacc ttgataccaa tacgtccagc tgacccagaa   720
aaaaatcaga gcttagttaa atcactgctg ctcaaggctg ttgtatctgg taacgctcga   780
aatggagttg cactcactgc cctggatcag gatcacgtcg cagtcctagg aagtccacta   840
gcagcttcaa aggaatgcct ctctgtatgg aacataaaat ttcaaacact acagacttca   900
aaagagttac cacaaggdac cagtggtcaa ctctggtatt atggagaaca tttgtttatg   960
ctacatggaa aatctctaac tgtgattcca tacaagtgtg aagtgtcatc attagcaggt  1020
gctcttggaa aactcaagca tagtcaagat ccaggaactc atgtcgtgtc ccattttgta  1080
aactgggaga cacctcaagg atgtggactt gggttccaga actcagagca gtcaagaaga  1140
attttaagga gacgaaaaat tgaagtgagt ttacagccag aggttccacc atccaaacaa  1200
cttttgtcaa ccataatgaa agattcagaa aaacacattg aagtagaagt acggaaattt  1260
ttggctctga agcagacacc tgactttcat actgtcattg gggacacagt aacaggactt  1320
ctggaaaggt gtaaagcaga accatcattt tatccccgga actgtctgat gcagcttatc  1380
caaacgcatg tgctttctta cagtttgtgc cccgacttaa tggagattgc cttaaaaaag  1440
aaagatgtac agttgttaca actctgtcta cagcagttcc ctgacattcc tgaatcagtc  1500
acctgtgctt gcttaaaaat tttcttgagc attggtgatg acagtcttca agaaacagat  1560
gttaatatgg agtcagtttt tgactatagt ataaattctg tacatgatga gaaaatggaa  1620
gagcaaactg aaattcttca aaatggcttc aatcctgaag aagataaatg caataactgt  1680

gatcaagagt taaataaaaa gccccaggac gaaacaaagg agagcacttc atgccctgtg 1740
gtacaaaaaa gagcagctct acttaatgca attcttcatt cagcatatag cgagacattt 1800
cttctgcctc atttgaaaga catcccagca cagcatatca cgctgtttct taagtatttg 1860
tatttcctgt acctgaagtg tagcgaaaat gctactatga ctcttcctgg aatacaccca 1920
cctaccttga accagattat ggattggata tgtctacttc tggatgcaaa ttttactgtt 1980
gttgtaatga tgccagaagc aaagaggcta ctgataaatc tttacaagct tgtaaaatct 2040
cagatatctg tttattctga gctcaacaag attgaagtaa gttttcggga gctacagaaa 2100
ttaaatcaag aaaagaataa tagaggatta tattcaattg aagtgctgga gctcttc 2157

<210> 40
<211> 20
<212> DNA
<213> Artificial Sequence

<220>
<223> Antisense oligonucleotide 4

<400> 40
gcattttcgc tacacttcag 20

<210> 41
<211> 20
<212> DNA
<213> Artificial Sequence

<220>
<223> Control oligonucleotide 4

<400> 41
gacttcacat cgcttttacg 20

<210> 42
<211> 21
<212> DNA
<213> Artificial Sequence

<220>

<223> Primer

<400> 42
tacagccaga ggttccacca t                                                21

<210> 43
<211> 22
<212> DNA
<213> Artificial Sequence

<220>
<223> Primer

<400> 43
tcctgttact gtgtccccaa tg                                               22

<210> 44
<211> 25
<212> DNA
<213> Artificial Sequence

<220>
<223> Primer

<400> 44
ctcaggcccg ttcattctcg cgaga                                            25

<210> 45
<211> 30
<212> DNA
<213> Artificial Sequence

<220>
<223> Primer

<400> 45
tgagatggtc gcaaacaaaa caccgttctt                                       30

<210> 46
<211> 21
<212> DNA
<213> Artificial Sequence

<220>
<223> Primer

<400> 46
ggatccatgg cagcgctgga g                                                21

<210> 47
<211> 27
<212> DNA
<213> Artificial Sequence

<220>
<223> Primer

<400> 47
ctcgagtcag aagagctcca gcacttc                                          27

<210> 48
<211> 715
<212> PRT
<213> Homo sapiens

<400> 48
Met Val Pro Gly Ala Arg Gly Gly Gly Ala Leu Ala Arg Ala Ala Gly
                5                   10                  15
Arg Gly Leu Leu Ala Leu Leu Leu Ala Val Ser Ala Pro Leu Arg Leu
            20                  25                  30
Gln Ala Glu Glu Leu Gly Asp Gly Cys Gly His Leu Val Thr Tyr Gln
        35                  40                  45
Asp Ser Gly Thr Met Thr Ser Lys Asn Tyr Pro Gly Thr Tyr Pro Asn
    50                  55                  60

His Thr Val Cys Glu Lys Thr Ile Thr Val Pro Lys Gly Lys Arg Leu
65                  70                  75                  80
Ile Leu Arg Leu Gly Asp Leu Asp Ile Glu Ser Gln Thr Cys Ala Ser
                85                  90                  95
Asp Tyr Leu Leu Phe Thr Ser Ser Ser Asp Gln Tyr Gly Pro Tyr Cys
            100                 105                 110
Gly Ser Met Thr Val Pro Lys Glu Leu Leu Leu Asn Thr Ser Glu Val
        115                 120                 125
Thr Val Arg Phe Glu Ser Gly Ser His Ile Ser Gly Arg Gly Phe Leu
    130                 135                 140
Leu Thr Tyr Ala Ser Ser Asp His Pro Asp Leu Ile Thr Cys Leu Glu
145                 150                 155                 160
Arg Ala Ser His Tyr Leu Lys Thr Glu Tyr Ser Lys Phe Cys Pro Ala
                165                 170                 175
Gly Cys Arg Asp Val Ala Gly Asp Ile Ser Gly Asn Met Val Asp Gly
            180                 185                 190
Tyr Arg Asp Thr Ser Leu Leu Cys Lys Ala Ala Ile His Ala Gly Ile
        195                 200                 205
Ile Ala Asp Glu Leu Gly Gly Gln Ile Ser Val Leu Gln Arg Lys Gly
    210                 215                 220
Ile Ser Arg Tyr Glu Gly Ile Leu Ala Asn Gly Val Leu Ser Arg Asp
225                 230                 235                 240
Gly Ser Leu Ser Asp Lys Arg Phe Leu Phe Thr Ser Asn Gly Cys Ser
                245                 250                 255
Arg Ser Leu Ser Phe Glu Pro Asp Gly Gln Ile Arg Ala Ser Ser Ser
            260                 265                 270
Trp Gln Ser Val Asn Glu Ser Gly Asp Gln Val His Trp Ser Pro Gly
        275                 280                 285
Gln Ala Arg Leu Gln Asp Gln Gly Pro Ser Trp Ala Ser Gly Asp Ser
    290                 295                 300
Ser Asn Asn His Lys Pro Arg Glu Trp Leu Glu Ile Asp Leu Gly Glu
305                 310                 315                 320
Lys Lys Lys Ile Thr Gly Ile Arg Thr Thr Gly Ser Thr Gln Ser Asn
                325                 330                 335
Phe Asn Phe Tyr Val Lys Ser Phe Val Met Asn Phe Lys Asn Asn Asn
            340                 345                 350
Ser Lys Trp Lys Thr Tyr Lys Gly Ile Val Asn Asn Glu Glu Lys Val

        355                 360                 365
Phe Gln Gly Asn Ser Asn Phe Arg Asp Pro Val Gln Asn Asn Phe Ile
    370                 375                 380
Pro Pro Ile Val Ala Arg Tyr Val Arg Val Val Pro Gln Thr Trp His
385                 390                 395                 400
Gln Arg Ile Ala Leu Lys Val Glu Leu Ile Gly Cys Gln Ile Thr Gln
                405                 410                 415
Gly Asn Asp Ser Leu Val Trp Arg Lys Thr Ser Gln Ser Thr Ser Val
            420                 425                 430
Ser Thr Lys Lys Glu Asp Glu Thr Ile Thr Arg Pro Ile Pro Ser Glu
        435                 440                 445
Glu Thr Ser Thr Gly Ile Asn Ile Thr Thr Val Ala Ile Pro Leu Val
    450                 455                 460
Leu Leu Val Val Leu Val Phe Ala Gly Met Gly Ile Phe Ala Ala Phe
465                 470                 475                 480
Arg Lys Lys Lys Lys Lys Gly Ser Pro Tyr Gly Ser Ala Glu Ala Gln
                485                 490                 495
Lys Thr Asp Cys Trp Lys Gln Ile Lys Tyr Pro Phe Ala Arg His Gln
            500                 505                 510
Ser Ala Glu Phe Thr Ile Ser Tyr Asp Asn Glu Lys Glu Met Thr Gln
        515                 520                 525
Lys Leu Asp Leu Ile Thr Ser Asp Met Ala Asp Tyr Gln Gln Pro Leu
    530                 535                 540
Met Ile Gly Thr Gly Thr Val Thr Arg Lys Gly Ser Thr Phe Arg Pro
545                 550                 555                 560
Met Asp Thr Asp Ala Glu Glu Ala Gly Val Ser Thr Asp Ala Gly Gly
                565                 570                 575
His Tyr Asp Cys Pro Gln Arg Ala Gly Arg His Glu Tyr Ala Leu Pro
            580                 585                 590
Leu Ala Pro Pro Glu Pro Glu Tyr Ala Thr Pro Ile Val Glu Arg His
        595                 600                 605
Val Leu Arg Ala His Thr Phe Ser Ala Gln Ser Gly Tyr Arg Val Pro
    610                 615                 620
Gly Pro Gln Pro Gly His Lys His Ser Leu Ser Ser Gly Gly Phe Ser
625                 630                 635                 640
Pro Val Ala Gly Val Gly Ala Gln Asp Gly Asp Tyr Gln Arg Pro His
                645                 650                 655

Ser Ala Gln Pro Ala Asp Arg Gly Tyr Asp Arg Pro Lys Ala Val Ser
            660                 665                 670
Ala Leu Ala Thr Glu Ser Gly His Pro Asp Ser Gln Lys Pro Pro Thr
        675                 680                 685
His Pro Gly Thr Ser Asp Ser Tyr Ser Ala Pro Arg Asp Cys Leu Thr
    690                 695                 700
Pro Leu Asn Gln Thr Ala Met Thr Ala Leu Leu
705                 710                 715

<210> 49
<211> 2145
<212> DNA
<213> Homo sapiens

<400> 49
atggtgcccg gcgcccgcgg cggcggcgca ctggcgcggg ctgccgggcg gggcctcctg    60
gctttgctgc tcgcggtctc cgccccgctc cggctgcagg cggaggagct gggtgatggc   120
tgtggacacc tagtgactta tcaggatagt ggcacaatga catctaagaa ttatcccggg   180
acctacccca atcacactgt ttgcgaaaag acaattacag taccaaaggg gaaaagactg   240
attctgaggt tgggagattt ggatatcgaa tcccagacct gtgcttctga ctatcttctc   300
ttcaccagct cttcagatca atatggtcca tactgtggaa gtatgactgt tcccaaagaa   360
ctcttgttga acacaagtga agtaaccgtc cgctttgaga gtggatccca catttctggc   420
cggggttttt tgctgaccta tgcgagcagc gaccatccag atttaataac atgtttggaa   480
cgagctagcc attatttgaa gacagaatac agcaaattct gcccagctgg ttgtagagac   540
gtagcaggag acatttctgg gaatatggta gatggatata gagatacctc tttattgtgc   600
aaagctgcca tccatgcagg aataattgct gatgaactag gtggccagat cagtgtgctt   660
cagcgcaaag ggatcagtcg atatgaaggg attctggcca atggtgttct ttcgagggat   720
ggttccctgt cagacaagcg atttctgttt acctccaatg gttgcagcag atccttgagt   780
tttgaacctg acgggcaaat cagagcttct tcctcatggc agtcggtcaa tgagagtgga   840
gaccaagttc actggtctcc tggccaagcc cgacttcagg accaaggccc atcatgggct   900
tcgggcgaca gtagcaacaa ccacaaacca cgagagtggc tggagatcga tttgggggag   960
aaaaagaaaa taacaggaat taggaccaca ggatctacac agtcgaactt caacttttat  1020
gttaagagtt ttgtgatgaa cttcaaaaac aataattcta agtggaagac ctataaagga  1080
attgtgaata atgaagaaaa ggtgtttcag ggtaactcta actttcggga cccagtgcaa  1140
aacaatttca tccctcccat cgtggccaga tatgtgcggg ttgtccccca gacatggcac  1200
cagaggatag ccttgaaggt ggagctcatt ggttgccaga ttacacaagg taatgattca  1260
ttggtgtggc gcaagacaag tcaaagcacc agtgtttcaa ctaagaaaga agatgagaca  1320

atcacaaggc ccatcccctc ggaagaaaca tccacaggaa taaacattac aacggtggct 1380
attccattgg tgctccttgt tgtcctggtg tttgctggaa tggggatctt tgcagccttt 1440
agaaagaaga agaagaaagg aagtccgtat ggatcagcag aggctcagaa aacagactgt 1500
tggaagcaga ttaaatatcc ctttgccaga catcagtcag ctgagtttac catcagctat 1560
gataatgaga aggagatgac acaaaagtta gatctcatca caagtgatat ggcagattac 1620
cagcagcccc tcatgattgg caccgggaca gtcacgagga agggctccac cttccggccc 1680
atggacacgg atgccgagga ggcaggggtg agcaccgatg ccggcggcca ctatgactgc 1740
ccgcagcggg ccggccgcca cgagtacgcg ctgcccctgg cgcccccgga gcccgagtac 1800
gccacgccca tcgtggagcg gcacgtgctg cgcgcccaca cgttctctgc gcagagcggc 1860
taccgcgtcc cagggcccca gcccggccac aaacactccc tctcctcggg cggcttctcc 1920
cccgtagcgg gtgtgggcgc ccaggacgga gactatcaaa ggccacacag cgcacagcct 1980
gcggacaggg gctacgaccg gcccaaagct gtcagcgccc tcgccaccga aagcggacac 2040
cctgactctc agaagccccc aacgcatccc gggacgagtg acagctattc tgcccccaga 2100
gactgcctca cacccctcaa ccagacggcc atgactgccc ttttg 2145

<210> 50
<211> 20
<212> DNA
<213> Artificial Sequence

<220>
<223> Antisense oligonucleotide 5

<400> 50
cgatacacac gcacactctg 20

<210> 51
<211> 20
<212> DNA
<213> Artificial Sequence

<220>
<223> Control oligonucleotide 5

<400> 51
gtctcacacg cacacatagc 20

<210> 52
<211> 20
<212> DNA
<213> Artificial Sequence

<220>
<223> Primer

<400> 52
cagtgtgctt cagcgcaaag                                                    20

<210> 53
<211> 20
<212> DNA
<213> Artificial Sequence

<220>
<223> Primer

<400> 53
   gcccgtcagg ttcaaaactc                                                   20

<210> 54
<211> 28
<212> DNA
<213> Artificial Sequence

<220>
<223> Primer

<400> 54
gtcgagggga ggccgagctt gccaagct                                           28

<210> 55
<211> 19
<212> RNA
<213> Artificial Sequence

<220>
<223> siRNA-1

<400> 55
acuacacuug cgaguuugc                                                    19

<210> 56
<211> 19
<212> RNA
<213> Artificial Sequence

<220>
<223> siRNA-1

<400> 56
gcaaacucgc aaguguagu                                                    19

<210> 57
<211> 19
<212> RNA
<213> Artificial Sequence

<220>
<223> siRNA-2

<400> 57
aaaguggagc augagagcu                                                    19

<210> 58
<211> 19
<212> RNA
<213> Artificial Sequence

<220>
<223> siRNA-2

<400> 58
agcucucaug cuccacuuu                                                    19

<210> 59
<211> 19
<212> RNA
<213> Artificial Sequence

<220>
<223> siRNA-3

<400> 59
ggucaucuuu guccgagaa                                                    19

<210> 60
<211> 19
<212> RNA
<213> Artificial Sequence

<220>
<223> siRNA-3

<400> 60
uucucggaca aagaugacc                                                    19

<210> 61
<211> 19
<212> RNA
<213> Artificial Sequence

<220>
<223> siRNA-4

<400> 61
gauucuacga uccgaaagc                                                    19

<210> 62

<211> 19
<212> RNA
<213> Artificial Sequence

<220>
<223> siRNA-4

<400> 62
gcuuucggau cguagaauc                                                    19

<210> 63
<211> 19
<212> RNA
<213> Artificial Sequence

<220>
<223> siRNA-5

<400> 63
ggaagagaag gcagagaaa                                                    19

<210> 64
<211> 19
<212> RNA
<213> Artificial Sequence

<220>
<223> siRNA-5

<400> 64
uuucucugcc uucucuucc                                                    19

<210> 65
<211> 15
<212> PRT
<213> Artificial Sequence

<220>
<223> Peptide 1 used in Example 16

<400> 65

<210> 66
<211> 15
<212> PRT
<213> Artificial Sequence

<220>
<223> Peptide 2 used in Example 16

<400> 66
Arg Glu Thr Pro Arg Ala Ser Pro Arg Asp Val Gly Pro Leu Cys
                5                   10                  15

<210> 67
<211> 15
<212> PRT
<213> Artificial Sequence

<220>
<223> Peptide 3 used in Example 16

<400> 67
Cys Thr Leu Gly Ala Ser Glu His Ser Pro Leu Lys Thr Pro Tyr
                5                   10                  15

<210> 68
<211> 25
<212> DNA
<213> Artificial Sequence

<220>

<223> Primer

<400> 68
aattgaattc atggcccggg ccgct                                          25

<210> 69
<211> 30
<212> DNA
<213> Artificial Sequence

<220>
<223> Primer

<400> 69
aattctcgag ggcccctgcg cctgctgtgt                                     30

<210> 70
<211> 371
<212> PRT
<213> Homo sapiens

<400> 70
Met Ala Arg Ala Ala Ala Leu Leu Pro Ser Arg Ser Pro Pro Thr Pro
                5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His

115                 120                 125
Gly Leu Thr Val Glu Asp Glu Gly Asn Tyr Thr Cys Glu Phe Ala Thr
    130                 135                 140
Phe Pro Lys Gly Ser Val Arg Gly Met Thr Trp Leu Arg Val Ile Ala
145                 150                 155                 160
Lys Pro Lys Asn Gln Ala Glu Ala Gln Lys Val Thr Phe Ser Gln Asp
                165                 170                 175
Pro Thr Thr Val Ala Leu Cys Ile Ser Lys Glu Gly Arg Pro Pro Ala
            180                 185                 190
Arg Ile Ser Trp Leu Ser Ser Leu Asp Trp Glu Ala Lys Glu Thr Gln
        195                 200                 205
Val Ser Gly Thr Leu Ala Gly Thr Val Thr Val Thr Ser Arg Phe Thr
    210                 215                 220
Leu Val Pro Ser Gly Arg Ala Asp Gly Val Thr Val Thr Cys Lys Val
225                 230                 235                 240
Glu His Glu Ser Phe Glu Glu Pro Ala Leu Ile Pro Val Thr Leu Ser
                245                 250                 255
Val Arg Tyr Pro Pro Glu Val Ser Ile Ser Gly Tyr Asp Asp Asn Trp
            260                 265                 270
Tyr Leu Gly Arg Thr Asp Ala Thr Leu Ser Cys Asp Val Arg Ser Asn
        275                 280                 285
Pro Glu Pro Thr Gly Tyr Asp Trp Ser Thr Thr Ser Gly Thr Phe Pro
    290                 295                 300
Thr Ser Ala Val Ala Gln Gly Ser Gln Leu Val Ile His Ala Val Asp
305                 310                 315                 320
Ser Leu Phe Asn Thr Thr Phe Val Cys Thr Val Thr Asn Ala Val Gly
                325                 330                 335
Met Gly Arg Ala Glu Gln Val Ile Phe Val Arg Glu Thr Pro Asn Thr
            340                 345                 350
Ala Gly Ala Gly Ala Thr Gly Gly Ile Leu Glu Asp Tyr Lys Asp Asp
        355                 360                 365
Asp Asp Lys
    370

<210> 71
<211> 1113
<212> DNA

<213> Homo sapiens

<400> 71
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatttccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agagaccccc aacacagcag gcgcaggggc cacaggcggc 1080
atcctcgagg attacaagga tgacgacgat aag 1113

## Description

### TECHNICAL FIELD

The present invention relates to an agent for the prevention/treatment or diagnosis of cancer, an agent for promoting apoptosis of cancer cells, an agent for inhibiting growth of cancer cells, an agent for inducing cell cycle change in cancer cells, screening of these agents, and so on.

### BACKGROUND ART

It is predicted that a cancer could be assessed for its pathological conditions by microarray profiling data for the gene. Actually in leukemia, it is reportedly possible to classify leukemia by gene expression profiles. By clarifying the gene expression profile of each cancerous tissue and accumulating its classification, it is considered possible to predict any response to a particular cancer therapy or discover a novel drug development target protein for a particular cancer. Specifically, where enhanced expression of a certain protein is observed in a certain cancer, it becomes possible to induce an anti-tumor activity in patients newly diagnosed to be antigen positive, by means of (i) reducing its expression level, (ii) suppressing its function, (iii) eliciting immune response of host to the protein, etc. At the same time, patients diagnosed to be antigen negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based drugs.

Nectin-2α gene (RefSeq Accession No. NM_002856) and Nectin-2δ gene (EMBL Accession No. X80038) are genes cloned from cDNA derived from human leukemia cell line TF-1, which encode proteins consisting of 479 amino acids and 538 amino acids, respectively (RefSeq Accession No. NP_002847 and EMBL Accession No. CAA56342). Nectin-2δ gene is a splicing variant of Nectin-2α gene; while the protein encoded by Nectin-2δ gene has an amino acid sequence corresponding to the 1st - 350th amino acid sequence in the protein encoded by Nectin-2α gene, the amino acid sequence is different in the amino acid sequence on and after the 351st amino acid at the C-terminal portion. In addition, mouse genes (GenBank Accession No. BC009088 and RefSeq Accession No. NM_008990) showing homology to Nectin-2α gene and Nectin-2δ gene are cloned from a library derived from mouse ES cells and encode proteins consisting of 467 amino acids and 530 amino acids, respectively (GenBank Accession No. AAH09088 and RefSeq Accession No. NP_033016). These mouse genes have about 72% and about 72% homologies to Nectin-2α gene and Nectin-2δ gene, respectively, in terms of base sequence and about 69% and about 73% homologies to Nectin-2α gene and Nectin-2δ gene, respectively, in terms of amino acid sequence. Nectin-2α and Nectin-2δ gene (hereinafter sometimes collectively referred to as Nectin-2) are also called as PVRL2, PRR2, PVRR2, HVEB, CD112, etc., and belong to the Nectin family. The Nectin family consists of four subfamilies, Nectin-1, Nectin-2, Nectin-3 and Nectin-4 (hereinafter they axe sometimes collectively referred to as Nectin). Nectins belong to the immunoglobulin superfamily and are single transmembrane type molecules having three immunoglobulin-like loops in the extracellular region. It is considered that Nectins would form *cis* dimers on the cell membranes and the *cis* dimers on the cell membrane at the juxtamembrane position *trans* interact with one another thereby to regulate cell-cell adhesion. The *trans* interaction of Nectin is induced by homophilic interaction with the same molecule, whereas Nectin-1 is known to be formed also heterophilically with Nectin-3 and Nectin-4, and Nectin-2 with Nectin-3. It is also known that Nectin binds to an actin-binding protein, afadin, through the intracellular C terminal region [J. Cell Sci. (2003), 116(1), 17-27]. Nectin is also considered to act as a receptor for glycoprotein D expressed on herpes simplex virus to function as a herpesvirus entry mediator in cells [J. Cell Sci. (2003), 116(1), 17-27]. Furthermore, it is considered that Nectin-2 would act as one of the ligands for DNAM-1 (CD226) expressed on NK cells and the DNAM-1-expressing NK cells would exert their cytotoxic activity, based on Nectin-2 expressed on target cells [J. Exp. Med. (2003), 198(4), 557-567]. On the other hand, Nectin-2 is reported to be one of the genes that take part in the p53 pathway of cancer suppressor genes (WO 02/99040). It is also reported that Nectin-2 is a protein binding to Nectin-3, which is a protein or useful for treating angiogenesis disorders, cancers or viral infections (WO 02/28902); a receptor engaged in viral infections (WO 99/63063); one of genes useful for the treatment and diagnosis for breast cancer or ovarian cancer (WO 02/00677); one of the 16 genes, which are overexpressed in various cancers and promising as a target for anti-tumor antibody medicines (WO 03/088808); and, one of the genes which are overexpressed in cancer tissues and promising for the diagnosis and prevention of cancer (WO 04/030615).

PSEC0110 fis gene (GenBank Accession No. AK075419) is a gene cloned from human placenta-derived cDNA and encodes a protein consisting of 341 amino acids (GenBank Accession No. BAC 11609, WO 00/05367, WO 00/11015, WO 01/77288, EP-A-1067182, etc.).

PHGDHL1 gene (RefSeq Accession No. NM_177967; EP-A-1067182) encodes a protein consisting of 307 amino acids (RefSeq Accession No. NP_808882), which protein has an amino acid sequence corresponding to the amino acid sequence 131st - 344th of a protein encoded by PSEC0110 fis gene wherein the 1st -130th amino acids are replaced. This PHGDHL1 gene has about 83% homology in its base sequence and about 74% homology in its amino acid sequence, to the PSEC0110 fis gene. In addition, a mouse gene (GenBank Accession No. AK049109) showing homology to the PSEC0110 fis gene is cloned from mouse ES cell-derived cDNA and encodes a protein consisting of 345 amino acids (GenBank Accession No. BAC33546). This mouse gene has about 85% homology in its base sequence and about 88% homology in its amino acid sequence, to the PSEC0110 fis gene.

KIAA0152 gene (RefSeq Accession No. NM_014730) is a gene cloned from human tissue-derived cDNA and encodes a protein consisting of 292 amino acids (RefSeq Accession No. NP_055545). Furthermore, a mouse gene (RefSeq Accession No. NM_175403) showing homology to the KIAA0152 gene is cloned from mouse tissue-derived cDNA and encodes a protein consisting of 291 amino acids (RefSeq Accession No. NP_780612). This mouse gene has about 87% homology in its base sequence and about 92% homology in its amino acid sequence, to the KIAA0152 gene. It is reported that the KIAA0152 gene is a gene useful for the diagnosis or treatment of liver cancer (WO 02/29103) and is one of the genes useful for screening of anti-cancer agents (WO 01/94629). Moreover, cloning of a human gene having 94% homology in its DNA sequence and 88% homology in its amino acid sequence is reported (WO 01/75067).

DKFZP586L0724 gene (RefSeq Accession No. NM_015462) is a gene (RefSeq Accession No. NP_056277) sequenced from human tissue-derived cDNA and encodes a protein consisting of 719 amino acids. Furthermore, a mouse gene (RefSeq Accession No. NM_133702) showing homology to the DKFZP586L0724 gene is cloned from mouse tissue-derived cDNA and encodes a protein consisting of 723 amino acids (RefSeq Accession No. NP_598463). This mouse gene has about 76% homology in its base sequence and about 74% homology in its amino acid sequence, to the DKFZP586L0724 gene. It is reported that the DKFZP586L0724 gene associated with ophthalmopathy such as retinal disorders, etc. (CN 1345826) and associated with metabolic disorders (CN 1345750).

DCBLD1L gene is a gene cloned from human T cell-derived cDNA and encodes a protein consisting of 715 amino acids (WO 01/29088, US 2003022279, WO 02/53739, etc.). The DCBLD1L gene (RefSeq Accession No. NM_173674) encodes a protein consisting of 539 amino acids (RefSeq Accession No. NP_775945). The DCBLD1 protein has an amino acid sequence corresponding to the 1st - 538th amino acid sequence in a protein encoded by the DCBLD1L gene but the 539th amino acid is replaced by glycine. In addition, a mouse gene (RefSeq Accession No. NM_025705) showing homology to the DCBLD1L gene (RefSeq Accession No. NP_079981) encodes a protein consisting of 503 amino acids. This mouse gene has about 53% homology in its base sequence and about 76% homology in its amino acid sequence, to the DCBLD1L gene. It is reported that the DCBLD1L is one of the genes useful for the diagnosis and treatment of ovarian cancer (WO 01/70979).

### DISCLOSURE OF THE INVENTION

A safe drug, which targets at a molecule specifically expressed in cancer cells to induce growth inhibition of cancer cells, has been earnestly desired.

The present inventors made extensive studies to solve the foregoing problems and as a result, have found a gene, expression of which is markedly enhanced in cancer tissues and also found that antisense oligonucleotide for this gene promotes apoptosis of cancer cells. Based on the findings, the inventors have continued further studies and come to accomplish the present invention.

That is, the present invention provides the following features and so on.
(1) An agent for preventing/treating cancer, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(2) An apoptosis promoter of cancer cells, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(3) A growth inhibitor of cancer cells, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(4) A diagnostic product for cancer, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(5) An agent for preventing/treating cancer, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(6) An apoptosis promoter of cancer cells, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(7) A growth inhibitor of cancer cells, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(8) An inducer of cell cycle change in cancer cells, which comprises (i) an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, (ii) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (iii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(9) An agent for preventing/treating cancer, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(10) An agent for preventing/treating cancer, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(11) An agent for preventing/treating cancer, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(12) An apoptosis promoter of cancer cells, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(13) An apoptosis promoter of cancer cells, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(14) An apoptosis promoter of cancer cells, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(15) A growth inhibitor of cancer cells, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(16) A growth inhibitor of cancer cells, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(17) A growth inhibitor of cancer cells, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.
(18) A diagnostic product for cancer, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(19) A method of screening an agent for preventing/treating cancer, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(20) A method of screening an agent for preventing/treating cancer, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(21) A method of screening an apoptosis promoter of cancer cells, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(22) A method of screening an apoptosis promoter of cancer cells, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(23) A method of screening a growth inhibitor of cancer cells, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(24) A method of screening a growth inhibitor of cancer cells, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(25) A kit for screening an agent for preventing/treating cancer, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(26) A kit for screening an agent for preventing/treating cancer, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(27) A kit for screening an apoptosis promoter of cancer cells, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(28) A kit for screening an apoptosis promoter of cancer cells, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(29) A kit for screening a growth inhibitor of cancer cells, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(30) A kit for screening a growth inhibitor of cancer cells, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.
(31) A method for preventing/treating cancer, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(32) A method for promoting apoptosis of cancer cells, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(33) A method for inhibiting growth of cancer cells, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(34) A method for preventing/treating cancer, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(35) A method for promoting apoptosis of cancer cells, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(36) A method for inhibiting growth of cancer cells, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.
(37) Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an agent for preventing/treating cancer.
(38) Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an apoptosis promoter of cancer cells.
(39) Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture a growth inhibitor of cancer cells.
(40) Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or a salt thereof, to manufacture an agent for preventing/treating cancer.
(41) Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an apoptosis promoter of cancer cells.
(42) Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture a growth inhibitor of cancer cells.

### BEST MODE FOR CARRYING OUT THE INVENTION

SEQ ID NO: 1, SEQ ID NO: 3, SEQ ID NO: 17, SEQ ID NO: 25, SEQ ID NO: 38 or SEQ ID NO: 48 (hereinafter these proteins are sometimes referred to as the protein of the present invention or as the protein used in the present invention) may be any protein derived from any cells of human and warm-blooded animals (e.g., guinea pig, rat, mouse, fowl, rabbit, swine, sheep, bovine, simian, etc.) such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence substantially identical to the same amino acid sequence as that represented by SEQ ID NO: 1, SEQ ID NO: 3, SEQ ID NO: 17, SEQ ID NO: 25, SEQ ID NO: 38 or SEQ ID NO: 48 includes amino acid sequences having at least about 50% homology, preferably at least about 60% homology, preferably at least about 70% homology, preferably at least about 80% homology, preferably at least about 90% homology and preferably at least about 95% homology, to the amino acid sequence shown by SEQ ID NO: 1, SEQ ID NO: 3, SEQ ID NO: 17, SEQ ID NO: 25, SEQ ID NO: 38 or SEQ ID NO: 48; etc.

Preferred examples of the protein comprising substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48 include proteins comprising substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48 and having a property substantially equivalent to that of the protein containing the amino acid sequence represented by SEQ ID NO: 1, SEQ ID NO: 3, SEQ ID NO: 17, SEQ ID NO: 25, SEQ ID NO: 38 or SEQ ID NO: 48, etc.

Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The substantially equivalent is used to mean that the nature of these properties is equivalent in terms of quality (e.g., physiologically or pharmacologically). Thus, the activity of protein used in the present invention is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

Examples of the protein used in the present invention include so-called muteins such as proteins having (i) the amino acid sequence represented by SEQ ID NO: 1, SEQ ID NO: 3, SEQ ID NO: 17, SEQ ID NO: 25, SEQ ID NO: 38 or SEQ ID NO: 48, of which at least 1 or 2 (e.g., about 1 to about 50, preferably about 1 to about 30, more preferably about 1 to about 10 and most preferably several (1 to 5)) amino acids are deleted; (ii) the amino acid sequence represented by SEQ ID NO: 1, SEQ ID NO: 3, SEQ ID NO: 17, SEQ ID NO: 25, SEQ ID NO: 38 or SEQ ID NO: 48, to which at least 1 or 2 (e.g., about 1 to about 50, preferably about 1 to about 30, more preferably about 1 to about 10 and most preferably several (1 to 5)) amino acids are added; (iii) the amino acid sequence represented by S SEQ ID NO: 1, SEQ ID NO: 3, SEQ ID NO: 17, SEQ ID NO: 25, SEQ ID NO: 38 or SEQ ID NO: 48, in which at least 1 or 2 (e.g., about 1 to about 50, preferably about 1 to about 30, more preferably about 1 to about 10 and most preferably several (1 to 5)) amino acids are inserted; (iv) the amino acid sequence represented by SEQ ID NO: 1, SEQ ID NO: 3, SEQ ID NO: 17, SEQ ID NO: 25, SEQ ID NO: 38 or SEQ ID NO: 48, in which at least 1 or 2 (e.g., about 1 to about 50, preferably about 1 to about 30, more preferably about 1 to about 10 and most preferably several (1 to 5)) amino acids are substituted by other amino acids; or (v) a combination of these amino acid sequences, which is so-called mutein; and the like.

Where the amino acid sequence is inserted, deleted or substituted as described above, the position of its insertion, deletion or substitution is not particularly limited.

Throughout the specification, the proteins are represented in accordance with the conventional way of describing peptides, that is, the N-terminus (amino terminus) at the left hand and the C-terminus (carboxyl terminus) at the right hand. In the protein used in the present invention including the protein comprising the amino acid sequence represented by SEQ ID NO: 1, the C-terminus may be in any form of a carboxyl group (-COOH), a carboxylate (-COO⁻), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention include a protein comprising the amino acid sequence represented by SEQ ID NO: 1, a protein comprising the amino acid sequence represented by SEQ ID NO: 3, a protein comprising the amino acid sequence represented by SEQ ID NO: 17, a protein comprising the amino acid sequence represented by SEQ ID NO: 25, a protein comprising the amino acid sequence represented by SEQ ID NO: 38, a protein comprising the amino acid sequence represented by SEQ ID NO: 48, and so on.

The partial peptide of the protein used in the present invention may be any peptide as long as it is a partial peptide of the protein used in the present invention described above and preferably has the property equivalent to that of the protein used in the present invention described above.

For example, there are used peptides containing, e.g., at least 20, preferably at least 50, more preferably at least 70, much more preferably at least 100 and most preferably at least 200 amino acids, in the constituent amino acid sequence of the protein used in the present invention, etc.

The partial peptide used in the present invention may be peptides containing the amino acid sequence, of which at least 1 or 2 (preferably about 1 to about 20, more preferably about 1 to about 10 and most preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 20, more preferably several and most preferably about 1 to about 5) amino acids may be substituted by other amino acids.

In the partial peptide used in the present invention, the C-terminus may be in any form of a carboxyl group (-COOH), a carboxylate (-COO⁻), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated peptides such as so-called glycopeptides having sugar chains; etc., as in the protein used in the present invention described above.

Specifically, there are employed:
(1) a peptide having the amino acid sequence represented by SEQ ID NO: 65 (Cys is added to the 88-101 amino acid sequence of the amino acid sequence represented by SEQ ID NO: 1 or 3 at its N terminus),
(2) a peptide having the amino acid sequence represented by SEQ ID NO: 66 (Cys is added to the 347-360 amino acid sequence of the amino acid sequence represented by SEQ ID NO: 1 at its C terminus),
(3) a peptide having the amino acid sequence represented by SEQ ID NO: 67 (Cys is added to the 426-439 amino acid sequence of the amino acid sequence represented by SEQ ID NO: 3 at its N terminus),
(4) an entire extracellular domain of a protein comprising the amino acid sequence represented by SEQ ID NO: 1 or SEQ ID NO: 3, or an immunogenic peptide (epitope) contained in said domain, and the like.

The length of such an immunogenic peptide is not particularly limited so long as it is long enough to have immunogenicity. The immunogenic peptide has consecutive amino acid residues of, for example, 8, preferably 10 and more preferably 12.

The partial peptide used in the present invention may also be used as an antigen for producing antibodies.

As salts of the protein or partial peptides used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or its partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or non-human mammalian tissues or cells are homogenized, extracted with an acid or the like, and the extract is purified and isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten After completion of the reaction, the partial peptide used in the present invention may be purified and isolated by a combination of conventional purification methods such as solvent extraction, distillation, column chromatography, liquid chromatography and recrystallization. When the partial peptide obtained by the above methods is in a free form, the partial peptide can be converted into an appropriate salt by a publicly known method or its modification; conversely when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Examples of the DNA encoding the protein used in the present invention may be any one of: a DNA comprising the base sequence represented by SEQ ID NO: 2, SEQ ID NO: 4, SEQ ID NO: 18, SEQ ID NO: 26, SEQ ID NO: 39 or SEQ ID NO: 49, or a DNA comprising a base sequence hybridizable to the base sequence represented by SEQ ID NO: 2, SEQ ID NO: 4, SEQ ID NO: 18, SEQ ID NO: 26, SEQ ID NO: 39 or SEQ ID NO: 49 under high stringent conditions and encoding a protein which has the properties of substantially the same nature as those of the protein comprising the amino acid sequence represented by SEQ ID NO: 1, SEQ ID NO: 3, SEQ ID NO: 17, SEQ ID NO: 25, SEQ ID NO: 38 or SEQ ID NO: 48.

As the DNA that is hybridizable to the base sequence represented by SEQ ID NO: 2, SEQ ID NO: 4, SEQ ID NO: 18, SEQ ID NO: 26, SEQ ID NO: 39 or SEQ ID NO: 49 under high stringent conditions, there are employed, for example, DNAs comprising base sequences having at least about 50% homology, preferably at least about 60% homology, preferably at least about 70% homology, preferably at least about 80% homology, preferably at least about 90% homology and preferably at least about 95% homology, to the base sequence represented by SEQ ID NO: 2, SEQ ID NO: 4, SEQ ID NO: 18, SEQ ID NO: 26, SEQ ID NO: 39 or SEQ ID NO: 49; and the like.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. More preferably, the hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, there are employed: (i) a DNA comprising the base sequence represented by SEQ ID NO: 2, a DNA comprising the base sequence represented by SEQ ID NO: 3, etc. as the DNA encoding the protein comprising the amino acid sequence represented by SEQ ID NO: 1; (ii) a DNA comprising the base sequence represented by SEQ ID NO: 4, etc. as the DNA encoding the protein comprising the amino acid sequence represented by SEQ ID NO: 3; (iii) a DNA comprising the base sequence represented by SEQ ID NO: 18, etc. as the DNA encoding the protein comprising the amino acid sequence represented by SEQ ID NO: 17; (iv) a DNA comprising the base sequence represented by SEQ ID NO: 26, etc. as the DNA encoding the protein comprising the amino acid sequence represented by SEQ ID NO: 25; (v) a DNA comprising the base sequence represented by SEQ ID NO: 39, etc. as the DNA encoding the protein comprising the amino acid sequence represented by SEQ ID NO: 38; (vi) a DNA comprising the base sequence represented by SEQ ID NO: 49, etc. as the DNA encoding the protein comprising the amino acid sequence represented by SEQ ID NO: 48.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, SEQ ID NO: 4, SEQ ID NO: 18, SEQ ID NO: 26, SEQ ID NO: 39 or SEQ ID NO: 49, or a DNA comprising a base sequence hybridizable to the base sequence represented by SEQ ID NO: 2, SEQ ID NO: 4, SEQ ID NO: 18, SEQ ID NO: 26, SEQ ID NO: 39 or SEQ ID NO: 49 under high stringent conditions and comprising a part of DNA encoding a protein having the activities of substantially the same nature as those of the protein of the present invention, and the like.

The DNA hybridizable to the base sequence represented by SEQ ID NO: 2, SEQ ID NO: 4, SEQ ID NO: 18, SEQ ID NO: 26, SEQ ID NO: 39 or SEQ ID NO: 49 indicates the same meaning as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the same and their expression), the DNA can be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (Takara Bio) or Mutan™-K (Takara Bio), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five^{™} cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include simian cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors bearing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium , [Bostian, K. L. et al., Proc. Natl. Acad. Sci. U.S.A. 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibody to the protein or partial peptide used in the present invention or its salts (hereinafter sometimes merely referred to as the antibody of the present invention) may be any of polyclonal and monoclonal antibodies so long as it recognizes the protein or partial peptide used in the present invention or its salts.

The antibody to the protein or partial peptide used in the present invention or its salts (hereinafter they are sometimes briefly referred to as the protein of the present invention) can be manufactured by publicly known methods for manufacturing antibodies or antisera.

Hereinafter, preparation of an antigen for the antibody of the present invention and preparation of the antibody will be described.

### (1) Preparation of antigen

As an antigen used to prepare the antibody of the present invention, any antigen such as a (synthetic) peptide having 1 or 2 more antigenic determinants, which are the same as in the protein comprising the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide or salts thereof, etc. may be used (hereinafter these antigens are sometimes merely referred to as the antigen of the present invention).

Specific examples of the antigen of the present invention include;
(1) a peptide having the amino acid sequence represented by SEQ ID NO: 65 (Cys is added to the 88-101 amino acid sequence of the amino acid sequence represented by SEQ ID NO: 1 or 3 at its N terminus),
(2) a peptide having the amino acid sequence represented by SEQ ID NO: 66 (Cys is added to the 347-360 amino acid sequence of the amino acid sequence represented by SEQ ID NO: 1 at its C terminus),
(3) a peptide having the amino acid sequence represented by SEQ ID NO: 67 (Cys is added to the 426-439 amino acid sequence of the amino acid sequence represented by SEQ ID NO: 3 at its N terminus),
(4) an entire extracellular domain of a protein comprising the amino acid sequence represented by SEQ ID NO: 1 or SEQ ID NO: 3, or an immunogenic peptide (epitope) contained in the domain, and the like.

The length of such an immunogenic peptide is not particularly limited so long as it is long enough to have immunogenicity. The immunogenic peptide has consecutive amino acid residues of, for example, 8, preferably 10 and more preferably 12.

The aforesaid protein, its partial peptide or salts thereof can be produced by publicly known methods. They may also be (a) prepared from tissues or cells of mammals such as human, simian, rat, mouse, etc., by publicly known methods or with modifications, (b) chemically synthesized by publicly known peptide synthesis methods using a peptide synthesizer, etc., or (c) produced by culturing a transformant bearing a DNA encoding a polypeptide comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof.
(a) Where the antigen of the present invention is prepared from the mammalian tissues or cells, the tissues or cells are homogenized, then a crude extract (ex., membrane fraction, soluble fraction) by itself can be used as an antigen. Alternatively, after extraction with an acid, a surfactant, an alcohol, etc., and the extract is purified and isolated by a combination of salting-out, dialysis, gel filtration, chromatography techniques such as reverse phase chromatography, ion exchange chromatography, affinity chromatography and the like.
(b) Where the antigen of the present invention is prepared chemically, the synthetic peptides used are, for example, a peptide having the same structure as the antigen of the present invention purified from natural one, a peptide containing 1 or 2 more amino acid sequences, which are the same amino acid sequences consisting of at least 3, preferably at least 6 amino acids in an optional region of the amino acid sequence represented by SEQ ID NO: 1, SEQ ID NO: 3, SEQ ID NO: 17, SEQ ID NO: 25, SEQ ID NO: 38 or SEQ ID NO: 48, etc.
(c) Where the protein comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof are produced using the DNA-bearing transformants, the DNA can be produced in accordance with publicly known cloning techniques [e.g., the method described in Molecular Cloning (2nd ed., J. Sambrook et al., Cold Spring Harbor Lab. Press, 1989), etc.]. The cloning techniques include (1) a method in which transformants containing DNAs encoding the protein comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof are obtained from cDNA library by hybridization using DNA probes or DNA primers designed based on the amino acid sequence of the protein comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof, or (2) a method in which transformants containing DNAs encoding the protein comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof are obtained by PCR using DNA primers designed based on the amino acid sequence of a polypeptide comprising the amino acid sequence represented by SEQ ID NO: 1, SEQ ID NO: 3, SEQ ID NO: 17, SEQ ID NO: 25, SEQ ID NO: 38 or SEQ ID NO: 48, or salts thereof, etc.

Mammalian cells which express the protein of the present invention can also be used directly as the antigen of the present invention. As the mammalian cells, there can be used the naturally occurring cells as described in (a) above, cells transformed by the methods as described in (c) above, etc. Hosts used for the transformation may be any cells as far as they are cells collected from human, simian, rat, mouse, hamster, etc. and preferably used are HEK293, COS7, CHO-K1, NIH3T3, Balb3T3, FM3A, L929, SP2/0, P3U1, B16, P388, or the like. Naturally occurring mammalian cells or transformed mammalian cells, which express the protein of the present invention, can be injected to immune animal as a suspension of the cells in a medium used for tissue culture (e.g., RPMI 1640) or buffer (e.g., Hanks' Balanced Salt Solution). Immunization may be done by any method, as long as it can stimulate antibody production, and preferably used are intravenous injection, intraperitoneal injection, intramuscular injection, subcutaneous injection, etc.

The peptides used as the antigen of the present invention can also be prepared (1) by peptide synthesis methods publicly known, or (2) by cleaving the protein comprising the amino acid sequence represented by SEQ ID NO: 1, SEQ ID NO: 3, SEQ ID NO: 17, SEQ ID NO: 25, SEQ ID NO: 38 or SEQ ID NO: 48 with an appropriate peptidase.

For the methods for peptide synthesis, for example, any of solid phase synthesis and liquid phase syntheses may be used. That is, the partial peptides or amino acids that can construct the peptide are condensed with the remaining part. Where the product contains protecting groups, these protecting groups are removed to give the desired peptide. Publicly known methods for condensation and removal of the protecting groups are methods described below.
(i) M. Bodanszky & M.A. Ondetti: Peptide Synthesis, Interscience Publishers, New York (1966)
(ii) Schroeder & Luebke: The Peptide, Academic Press, New York (1965)

After completion of the reaction, the peptide may be purified and isolated by a combination of conventional purification methods such as solvent extraction, distillation, column chromatography, liquid chromatography and recrystallization. When the peptide obtained by the above methods is in a free form, the peptide can be converted into an appropriate salt by a publicly known method or its modification; conversely when the peptide is obtained in a salt form, it can be converted into a free form by a publicly known method.

To synthesize amides of the peptide, commercially available resins for peptide synthesis that are suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective peptide according to various condensation methods publicly known. At the end of the reaction, the peptide is excised from the resin and at the same time, the protecting groups are removed to obtain the objective peptide. Alternatively, the partially protected peptide may be taken out using chlorotrityl resin, oxime resin, 4-hydroxybenzoic acid resin, etc., the protective groups are removed in a conventional manner to obtain the objective peptide.

For condensation of the protected amino acids described above, a variety of activation reagents for peptide synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization-suppressing additive (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be appropriately chosen from solvents that are known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the amino groups in the starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups for carboxyl groups include a C₁₋₆ alkyl group, C₃₋₈ cycloalkyl group, a C₇₋₁₄ aralkyl group, 2-adamantyl, 4-nitrobenzyl, 4-methoxybenzyl, 4-chlorobenzyl, phenacyl, benzyloxycarbonyl hydrazide, trityl hydrazide, or the like.

The hydroxyl group of serine and threonine can be protected through, for example, its esterification or etherification. Examples of the groups suitable for the esterification include a lower (C₁₋₆) alkanoyl group, such as acetyl group, etc.; an aroyl group such as benzoyl group, etc., and a group derived from carbonic acid such as benzyloxycarbonyl group, ethoxycarbonyl group, etc. Examples of a group suitable for the etherification include benzyl group, tetrahydropyranyl group, t-butyl group, etc.

Examples of groups for protecting the phenolic hydroxyl group of tyrosine include Bzl, Cl-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, Bom, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrofluoric acid, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting groups by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups and activation of the functional groups involved in the reaction may be appropriately chosen from publicly known groups and publicly known means.

In another method for obtaining the amides of the peptide, for example, the α-carboxyl group of the carboxy terminal amino acid is first protected by amidation; the peptide chain is then extended to a desired length toward the amino group side. Thereafter, a peptide in which only the protecting group of the N-terminal α-amino group in the peptide chain has been eliminated from the peptide and a peptide (or amino acids) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means. Lyophilization of the major fraction gives the amide of the desired peptide.

To prepare the esterified peptide, for example, the α-carboxyl group of the carboxy terminal amino acid is condensed with a desired alcohol to prepare the amino acid ester, which is followed by procedure similar to the preparation of the amidated peptide above to give the ester form of the desired peptide.

The antigen of the present invention may be provided for direct immunization in its immobilized form. The antigen of the present invention may also be bound or adsorbed to an appropriate carrier and the complex produced can be provided for immunization. A mixing ratio of the carrier to the antigen of the present invention (hapten) may be in any ratio of any type, as long as the antibody can be efficiently produced to the antigen of the present invention. A high molecular carrier conventionally used to produce an antibody to a hapten may be used in a weight ratio of 0.1 to 100 based on 1 of hapten. As such a high molecular carrier, there are used a naturally occurring high molecular carrier and a synthetic high molecular carrier. Examples of the naturally occurring high molecular carrier used are serum albumin from mammals such as bovine, rabbit, human, etc., thyroglobulins from mammals such as bovine, rabbit, etc., hemoglobins from mammals such as bovine, rabbit, human, sheep, etc or keyhole limpet KHL hemocyanin. Examples of the synthetic high molecular carrier, which can be used, are various latexes including polymers, copolymers, etc., for example, polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiols with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent (e.g., N-succinimidyl 3-(2-pyridyldithio)propionate (SPDP), etc.) having dithiopyridyl group and then reduced to introduce the thiol group, whereas another amino group is introduced with a maleimide group using a maleimide activated ester reagent, and the two groups may be reacted with each other.

### [Preparation of monoclonal antibody]

### (a) Preparation of monoclonal antibody-producing cells

The antigen of the present invention is administered to warm-blooded animals. The site to be administered may be a site where the production of antibody is possible, and administration is performed either solely or together with carriers or diluents. In order to potentiate the antibody productivity upon the administration, complete Freund's adjuvants or incomplete Freund's adjuvants may be administered. The administration is usually carried out once every 2 to 6 weeks and 2 to 10 times in total. Further in preparing the monoclonal antibody of the present invention, DNA immunization may be used (see, e.g., Nature, 356, 152-154). Examples of the applicable warm-blooded animals are simian, rabbits, canine, guinea pigs, mice, rats, sheep, goats and fowl, with the use of mice and rats being preferred for preparation of the monoclonal antibody.

In preparation of the monoclonal antibody-producing cells, a warm-blooded animal, e.g., mice, immunized with the antigen of the present invention wherein the antibody titer is noted is selected, then spleen or lymph node is collected after 2 to 5 days from the final immunization and antibody-producing cells contained therein are fused with myeloma cells to give hybridomas producing the monoclonal antibody. Measurement of antibody titer of the antibody in antisera may be carried out, for example, by reacting the labeled protein described later with the antiserum followed by assaying the binding activity of the labeling agent bound to the antibody. The fusion may be carried out, for example, by the known method by Koehler and Milstein [Nature, 256, 495 (1975)]. Examples of the fusion accelerator are polyethylene glycol (PEG), Sendai virus, etc., of which PEG is preferably employed.

Examples of the myeloma cells are those collected from warm-blooded animals such asNS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of hybridomas producing the monoclonal antibody. Examples of such methods include a method which comprises adding the culture supernatant of a hybridoma to a solid phase (e.g., a microplate) adsorbed with the protein antigen directly or together with a carrier, adding an anti-immunoglobulin antibody (where mouse cells are used for the cell fusion, anti-mouse immunoglobulin antibody is used) labeled with a radioisotope or enzyme, or Protein A and detecting the monoclonal antibody bound to the solid phase, a method which comprises adding the culture supernatant of a hybridoma to a solid phase adsorbed with an anti-immunoglobulin antibody or Protein A, adding a protein labeled with a radioisotope or enzyme and detecting the monoclonal antibody bound to the solid phase, and the like.

The screening of the monoclonal antibody can be performed in a medium for animal cells normally supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for incubation of a hybridoma (SFM-101, Nissui Seiyaku Co., Ltd.) and the like, can be used for the screening and growth medium. The culture is carried out generally at a temperature of 20 to 40°C, preferably at 37°C, for about 5 days to about 3 weeks, preferably 1 to 2 weeks. Incubation can be made normally in 5% carbon dioxide gas. Antibody titer of supernatant of the hybridoma culture can be measured with similar method to that of the antibody titer in the antiserum described above.

### (b) Preparation of monoclonal antibody

Separation and purification of the monoclonal antibody can be made by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody alone.]

### [Preparation of polyclonal antibody]

The polyclonal antibody of the present invention can be manufactured by publicly known methods or modifications thereof. For example, an immunogen (protein antigen) per se, a warm-blooded animal is immunized with an immunogen per se, or with a complex of immunogen and a carrier protein formed in a manner similar to the method for the manufacture of monoclonal antibodies described above. The product containing the antibody of the present invention is collected from the immunized animal followed by separation and purification of the antibody.

In the complex of immunogen and carrier protein used to immunize a warm-blooded animal, the type of carrier protein and the mixing ratio of carrier protein to hapten may be any type and in any ratio, as long as the antibody is efficiently produced to the hapten immunized by crosslinking to the carrier protein. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier protein to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total. Further in preparing the monoclonal antibody of the present invention, DNA immunization may be used (e.g., see Nature, 356, 152-154).

The polyclonal antibody can be collected from the blood, ascites, etc., preferably from the blood of warm-blooded animal immunized by the method described above.

The polyclonal antibody titer in antiserum can be assayed, for example, by the same procedure as the assay of antibody titer of the hybridoma culture supernatant described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, and antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, SEQ ID NO: 4, SEQ ID NO: 18, SEQ ID NO: 26, SEQ ID NO: 39 or SEQ ID NO: 49, preferably an antisense polynucleotide containing the entire or part of a base sequence complementary to a base sequence of DNA containing the base sequence represented by SEQ ID NO: 2, SEQ ID NO: 4, SEQ ID NO: 18, SEQ ID NO: 26, SEQ ID NO: 39, SEQ ID NO: 49, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2, SEQ iD NO: 4, SEQ ID NO: 18, SEQ ID NO: 26, SEQ ID NO: 39 or SEQ ID NO: 49. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such an antisense polynucleotide is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating/controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and proteins usually refer to amino acids of a protein (under the order) derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the target nucleic acids and the polynucleotides complementary to, and hybridizable to, at least a part of the target region, can be denoted to be "antisense" to the polynucleotides in the target region. Examples of the antisense polynucleotides include polydeoxyribonucleotides containing 2-deoxy-D-ribose, polyribonucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotide may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like. The antisense polynucleotide of the present invention is RNA, DNA or a modified nucleic acid (RNA, DNA). Specific examples of the modified nucleic acid include sulfur and thiophosphate derivatives of nucleic acids and those resistant to degradation such as polynucleoside amides or oligonucleoside amides. The antisense polynucleotide of the present invention can be designed as follows, that is, by increasing the intracellular stability of the antisense polynucleotide, enhancing the cell permeability of the antisense polynucleotide, increasing the affinity of the nucleic acid to the targeted sense strand to a higher level, or minimizing the toxicity, if any, of the antisense polynucleotide. Most of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibition activity of the antisense polynucleotide can be investigated using the transformant of the present invention, the in vivo or in vitro gene expression system of the present invention, or the in vivo or in vitro translation system of the protein of the present invention.

Applications of the protein or partial peptide of the present invention, or salts thereof (hereinafter sometimes simply referred to as the protein of the present inventidon), the DNA encoding the protein or partial peptide of the present invention (hereinafter sometimes simply referred to as the DNA of the present invention), the antibody to the protein or partial peptide of the present invention (hereinafter sometimes simply referred to as the antibody of the present invention) and the antisense polynucleotide of the DNA of the present invention (hereinafter sometimes simply referred to as the antisense polynucleotide of the present invention) are described below.

The protein of the present invention is increasingly expressed in cancer tissues and can be utilized as a disease marker. That is, the protein is useful as a marker for early diagnosis in cancer tissues, for judgment of severity in conditions, or for predicted development of these diseases. Therefore, the pharmaceuticals comprising the antisense polynucleotide to the polynucleotide encoding the protein of the present invention, the compound or its salt that inhibits the activity of the protein of the present invention, the compound or its salt that inhibits the expression of a gene for the protein of the present invention, or the antibody to the protein of the present invention can be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

### (1) Pharmaceutical comprising the antibody of the present invention

The antibody of the present invention, especially the antibody which recognizes (i) a peptide having the amino acid sequence represented by SEQ ID NO: 65 (Cys is added to the 88-101 amino acid sequence of the amino acid sequence represented by SEQ ID NO: 1 or 3 at its N terminus), (ii) a peptide having the amino acid sequence represented by SEQ ID NO: 66 (Cys is added to the 347-360 amino acid sequence of the amino acid sequence represented by SEQ ID NO: 1 at its C terminus), (iii) a peptide having the amino acid sequence represented by SEQ ID NO: 67 (Cys is added to the 426-439 amino acid sequence of the amino acid sequence represented by SEQ ID NO: 3 at its N terminus), or (iv) an entire extracellular domain of a protein comprising the amino acid sequence represented by SEQ ID NO: 1 or SEQ ID NO: 3, or an immunogenic peptide (epitope) contained in the domain, etc., has the activity of inducing/promoting the apoptosis of cancer cells, the activity of inhibiting cancer cell growth, etc. Therefore, the antibody can be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

The aforesaid agent for preventing/treating cancer, apoptosis promoter in cancer cells, cancer cell growth inhibitor and inducer of cell cycle change in cancer cells, which comprises the antibody of the present invention, are low-toxic and can be administered as they are in the form of liquid preparations, or as pharmaceutical compositions of suitable preparations to human or mammals (e.g., rats, rabbits, sheep, swine, bovine, feline, canine, simian, etc.) orally or parenterally (e.g., intravascularly, intraperitoneally, subcutaneously, etc.).

The antibody of the present invention may be administered in itself, or may be administered as an appropriate pharmaceutical composition. The pharmaceutical composition used for the administration may contain a pharmacologically acceptable carrier with the antibody of the present invention or its salt, a diluent or excipient. Such a pharmaceutical composition is provided in the form of pharmaceutical preparations suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the antibody of the present invention or its salt in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the antibody of the present invention or its salt with conventional bases for suppositories.

The composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a vehicle, a diluent or excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the antibody described above is contained in about 5 to about 100 mg especially in the form of injection, and in 10 to 250 mg for the other forms.

The dose of the aforesaid prophylactic/therapeutic agent or regulator comprising the antibody of the present invention may vary depending upon subject to be administered, target disease, conditions, route of administration, etc. For example, when used for the purpose of treating/preventing, e.g., breast cancer in adult, it is advantageous to administer the antibody of the present invention intravenously in a dose of about 0.01 to about 20 mg/kg body weight, preferably about 0.1 to about 10 mg/kg body weight and more preferably about 0.1 to about 5 mg/kg body weight, about 1 to 5 times/day, preferably about 1 to 3 times/day. In other parenteral and oral administration, the agent can be administered in a dose corresponding to the dose given above. When the condition is especially severe, the dose may be increased according to the condition.

The antibody of the present invention may be administered as it stands or in the form of an appropriate pharmaceutical composition. The pharmaceutical composition used for the administration may contain a pharmacologically acceptable carrier with the aforesaid antibody or its salts, a diluent or excipient. Such a composition is provided in the form of pharmaceutical preparations suitable for oral or parenteral administration (e.g., intravascular injection, subcutaneous injection, etc.).

Each composition described above may further contain other active components unless formulation causes any adverse interaction with the antibody described above.

Furthermore, the antibody of the present invention may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, irinotecan, etc. The antibody of the present invention and the drugs described above may be administered simultaneously or at staggered times to the patient.

### (2) Pharmaceutical comprising the antisense polynucleotide

The antisense polynucleotide of the present invention that binds to the DNA of the present invention complementarily to suppress the expression of said DNA is low toxic and can suppress the functions or effects of the protein of the present invention or the DNA of the present invention in vivo, induce the apoptosis of cancer cells, or inhibit the growth of cancer cells. Thus, the antisense polynucleotide can be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

Where the antisense polynucleotide described above is used as the aforesaid prophylactic/therapeutic agent, apoptosis promoter, growth inhibitor, inducer of cell cycle change in cancer cells, etc., the antisense polynucleotide can be prepared into a pharmaceutical preparation and provided for administration.

For example, the antisense polynucleotide itself, or the antisense polynucleotide inserted into an appropriate vector such as retrovirus vector, adenovirus vector, adenovirus-associated virus vector, etc., is administered orally or parenterally to human or mammal (e.g., rat, rabbit, sheep, swine, bovine, feline, canine, simian, etc.) in a conventional manner. The antisense polynucleotide may also be administered as it is, or prepared into medicines together with physiologically acceptable carriers such as adjuvants to assist its uptake, and such preparations are administered by gene gun or through a catheter like a hydrogel catheter. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to about 100 mg.

In addition, the antisense polynucleotide may also be used as an oligonucleotide probe for diagnosis to examine the presence of the DNA of the present invention in tissues or cells and states of its expression.

As the antisense polynucleotide described above can, the double-stranded RNA (siRNA) comprising a part of RNA encoding the protein of the present invention, ribozyme comprising a part of RNA encoding the protein of the present invention, etc. can also prevent expression of the gene of the present invention to suppress the in vivo function of the protein used in the present invention or the DNA used in the present invention and thus can be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and the like.

The double-stranded RNA can be designed based on the sequence of the polynucleotide of the present invention and manufactured by modifications of publicly known methods (e.g., Nature, 411, 494, 2001).

Examples of the double-stranded RNAs of the present invention used include the following RNAs prepared in EXAMPLE 11 later described, and so on.
(i) siRNA-1 wherein RNA having the base sequence represented by SEQ ID NO: 55 is hybridized to RNA having the base sequence represented by SEQ ID NO: 56,
(ii) siRNA-2 wherein RNA having the base sequence represented by SEQ ID NO: 57 is hybridized to RNA having the base sequence represented by SEQ ID NO: 58,
(iii) siRNA-3 wherein RNA having the base sequence represented by SEQ ID NO: 59 is hybridized to RNA having the base sequence represented by SEQ ID NO: 60,
(iv) siRNA-4 wherein RNA having the base sequence represented by SEQ ID NO: 61 is hybridized to RNA having the base sequence represented by SEQ ID NO: 62, and,
(v) siRNA-5 wherein RNA having the base sequence represented by SEQ ID NO: 63 is hybridized to RNA having the base sequence represented by SEQ ID NO: 64.

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the agents described above, the double-stranded RNA or ribozyme is prepared into a pharmaceutical preparation as in the antisense polynucleotide, and the preparation can be provided for administration.

### (3) Screening of drug candidate compounds for disease

Since the protein of the present invention is increasingly expressed in cancer tissues and when the activity of the protein of the present invention is inhibited, the cancer cells cause apoptosis to inhibit growth of the cancer cells. Accordingly, the compound or its salt that inhibits the activity of the protein of the present invention can be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

Accordingly, the protein of the present invention is useful as a reagent for screening the compound or its salts that inhibit the activity of the protein of the present invention.

That is, the present invention provides a method of screening the compound or its salts that inhibit the activity of the protein of the present invention, which comprises using the protein of the present invention.

Specifically, there is employed the method of screening the compound or its salts that inhibits the activity of the proteinof the present invention, which comprises comparing (i) the activity of a cell capable of producing the protein of the present invention with (ii) the activity of a mixture of the cell capable of producing the protein of the present invention and a test compound.

As the cells capable of producing the protein of the present invention described above, there are used, for example, the aforesaid host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been expressed in the cells, e.g., by culturing through the procedure described above, is preferably employed. The procedure for incubating the cells capable of expressing the protein of the present invention is similar to the incubation procedure for the transformant of the present invention described above.

Examples of the test compound include peptides, proteins, non-peptide compounds, synthetic compounds, fermentation products, cell extracts, plant extracts, animal tissue extracts, etc.

For example, when a test compound inhibits the activity of the protein of the present invention in the case (ii) described above by at least about 20%, preferably at least 30% and more preferably at least about 50%, as compared to the case (i) above, the test compound can be selected as the compound that inhibits the activity of the protein of the present invention.

The compound having the activity of inhibiting the activity of the protein of the present invention is useful as a safe and low toxic pharmaceutical for suppressing the physiological activities of the protein of the present invention.

Furthermore, the gene for the protein of the present invention also shows an increased expression in cancer tissues. Accordingly, the compound or its salts that inhibits the expression of the gene for the protein of the present invention can also be used as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

Therefore, the polynucleotide (e.g., DNA) of the present invention is useful as a reagent for screening the compound or its salts that inhibits the expression of the gene for the protein of the present invention.

For the screening method, there is a method of screening which comprises comparing (iii) the case where a cell capable of producing the protein of the present invention is incubated and (iv) the case where a cell capable of producing the protein used in the present invention is incubated in the presence of a test compound.

In the screening method described above, the expression level of the gene described above (specifically, the level of the protein of the present invention or the level of mRNA encoding said protein) is determined, followed by comparison between the cases (iii) and (iv).

Examples of the test compound and the cells capable of producing the protein of the present invention are the same as described above.

The level of the protein can be determined by publicly known methods, e.g., by measuring the aforesaid protein present in the cell extract, etc., using the antibody capable of recognizing the protein of the present invention, in accordance with methods such as western blot analysis, ELISA, etc., or their modifications.

The mRNA level can be determined by publicly known methods, e.g., in accordance with methods such as Northern hybridization using a nucleic acid containing the entire or a part of SEQ ID NO: 2, SEQ ID NO: 4, SEQ ID NO: 18, SEQ ID NO: 26, SEQ ID NO: 39 or SEQ ID NO: 49 as a probe, or PCR using a nucleic acid containing the entire or a part of SEQ ID NO: 2, SEQ ID NO: 4, SEQ ID NO: 18, SEQ ID NO: 26, SEQ ID NO: 39 or SEQ ID NO: 49 as a primer, or modifications thereof.

For example, when a test compound inhibits the expression of the gene in the case (iv) described above by at least about 20%, preferably at least 30% and more preferably at least about 50%, as compared to the case (iii) above, the test compound can be selected to be a compound inhibiting the expression of the gene for the protein of the present invention.

The screening kit of the present invention comprises the protein or its partial peptide used in the present invention or salts thereof, or a cell capable of producing the protein or its partial peptide used in the present invention.

The compound or its salt obtained by using the screening method or screening kit of the present invention is a compound or its salt, which is selected from the test compound described above, e.g., peptides, proteins, non-peptide compounds, synthetic compounds, fermentation products, cell extracts, plant extracts, animal tissue extracts, plasma, etc., or is a compound or its salt that inhibits the activity of the protein of the present invention, or is a compound or its salt that inhibits the expression of the gene for the protein of the present invention.

The salts used are those given above as the salts of the protein of the present invention.

The compound or its salt that inhibits the activity of the protein of the present invention and the compound or its salt that inhibits the expression of the gene for the protein of the present invention are useful as low-toxic and safe pharmaceuticals, respectively, such as an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), (preferably an agent for preventing/treating breast cancer, lung cancer, colon cancer, prostate cancer, ovary cancer, pancreatic cancer, etc.), an apoptosis promoter in cancer cells, a cancer cell growth inhibitor, an inducer of cell cycle change in cancer cells, and so on.

Where the compound or its salt obtained by using the screening method or screening kit of the present invention is used as the aforesaid prophylactic/therapeutic agent, it can be prepared into pharmaceutical preparations by publicly known methods.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the compound or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the compound or its salt described above with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the compound described above is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, the preparations can be administered to human or warm-blooded animal (e.g., mouse, rat, rabbit, sheep, swine, bovine, horse, fowl, feline, canine, simian, chimpanzee, etc.) orally or parenterally.

The dose of said compound or its salt may vary depending upon its effects, target disease, subject to be administered, route of administration, etc. For example, when the compound or its salt that inhibits the expression of the gene for the protein of the present invention is orally administered for the purpose of treating, e.g., breast cancer in adult (as 60 kg body weight), it is advantageous to administer the compound or its salt in a dose of about 0.1 to about 100 mg/day, preferably about 1.0 to about 50 mg/day and more preferably about 1.0 to about 20 mg/day. In parenteral administration, a single dose of said compound or its salt may also vary depending upon subject to be administered, target disease, etc. When the compound or its salt that inhibits the expression of the gene for the protein of the present invention is administered to an adult (as 60 kg body weight) in the form of an injectable preparation for the purpose of treating, e.g., breast cancer, it is advantageous to administer the compound or its salt at cancerous lesions by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

### (4) Quantification for the protein used in the present invention

The antibody of the present invention is capable of specifically recognizing the protein of the present invention and therefore can be used for quantification of the protein of the present invention in a test sample fluid, in particular, for quantification by sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention, or detect the protein by means of a tissue staining, etc. For these purposes, the antibody molecule per se may be used or F (ab')₂, Fab' or Fab fractions of the antibody molecule may also be used.

The quantification method using the antibody of the present invention is not particularly limited. Any quantification method may be used, so long as the amount of an antibody, antigen or antibody-antigen complex corresponding to the amount of antigen (e.g., the amount of the protein of the present invention) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is preferred to use the sandwich method and the competitive method later described, particularly the sandwich method.

Examples of labeling agents, which are employed for the assay methods using labeling agents, are radioisotopes, enzymes, fluorescent substances, luminescent substances, etc. Examples of radioisotopes include [¹²⁵I], [¹³¹I], [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include cyanine fluorescent dyes (e.g., Cy2, Cy3, Cy5, Cy5.5, Cy7 (manufactured by Amersham Biosciences), etc.), fluorescamine, fluorescein isothiocyanate, etc. Examples of the luminescent substances are luminol, a luminol derivative, luciferin, lucigenin, etc. Furthermore, a biotin-avidin system may be used as well for binding an antibody or antigen to a labeling agent.

In the immobilization of antigens or antibodies, physical adsorption may be used. Alternatively, chemical binding that is conventionally used for immobilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the method for determining the protein of the present invention by the sandwich method, the monoclonal antibodies of the present invention used for the primary and secondary reactions are preferably antibodies having sites different from one another, to which the protein of the present invention bind. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) And the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc. may be referred.

As described above, the protein of the present invention can be quantified with high sensitivity, using the antibody of the present invention.

Furthermore, when an increased level of the protein of the present invention is detected by quantifying the level of the protein of the present invention using the antibody of the present invention, it can be diagnosed that one suffers from cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or it is highly likely to suffer from these diseases in the future.

Moreover, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (5) Gene diagnostic product

By using the DNA of the present invention, e.g., as a probe, an abnormality (gene abnormality) of the DNA or mRNA encoding the protein or its partial peptide of the present invention in human or warm-blooded animal (e.g., rat, mouse, guinea pig, rabbit, fowl, sheep, swine, bovine, horse, feline, canine, simian, chimpanzee, etc.) can be detected. Therefore, the DNA is useful as a gene diagnostic product for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., Northern hybridization or DNA mutation is detected by the PCR-SSCP assay, it can be diagnosed that it is highly likely to suffer from, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

### (6) Pharmaceutical comprising the protein of the present invention

Since the protein of the present invention is overexpressed in cancer, the protein of the present invention can be used as a cancer vaccine to activate the immune system in patients with cancer.

For example, the so-called adoptive immunotherapy, which involves culturing potent antigen presenting cells (e.g., dendritic cells) in the presence of the protein of the present invention to engulf the protein and putting the cells back into the body, can preferably be used. The dendritic cells, returned back into the body, can induce and activate cytotoxic T cells specific to a cancer antigen whereby to kill cancer cells.

The protein of the present invention can also be administered to a mammal (e.g. human, simian, mouse, rat, rabbit, swine) safely as a vaccine preparation to prevent or treat a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.)

The vaccine preparation usually contains the protein of the present invention and a physiologically acceptable carrier. Such a carrier includes a liquid carrier such as water, saline (including physiological saline), buffer (e.g., phosphate buffer), an alcohol (e.g., ethanol), etc.

The vaccine preparation can be prepared according to a conventional method of manufacturing a vaccine preparation.

In general, the protein of the present invention is dissolved or suspended in a physiologically acceptable carrier. Alternatively, the protein of the present invention and the physiologically acceptable carrier may be separately prepared and then mixed at use.

The vaccine preparation may be further formulated with, for example, an adjuvant (e.g., aluminum hydroxide gel, serum albumin, etc.), a preservative (e.g., thimerosal, etc.), a soothing agent (e.g., glucose, benzyl alcohol, etc.), in addition to the protein of the present invention and the physiologically acceptable carrier. Furthermore, the vaccine preparation may also be formulated with, for example, a cytokine (e.g., an interleukin such as interleukin-2, an interferon such as interferon-γ) to enhance the production of the antibody to the protein of the present invention.

When used as a vaccine preparation, the protein of the present invention may be used in its active form, or the protein of the present invention may be denatured to enhance the antigenicity. The protein of the present invention may be denatured usually by heating or treating with a protein-denaturing agent (e.g., formalin, guanidine hydrochloride and urea).

The thus obtained vaccine preparation is low toxic and may usually be administered in an injectable form, e.g., subcutaneously, intracutaneously, intramuscularly, or topically into or near a mass of cancer cells.

The dose of the protein of the present invention varies depending on target disease, subject to be administered, route for administration, etc. For example, for subcutaneous administration of the protein of the present invention to an adult with cancer (60 kg body weight) in an injectable form, the single dose is normally about 0.1 mg to about 300 mg, preferably about 100 mg to about 300 mg. The administration of the vaccine preparation may be carried out once, or 2 to 4 times in total approximately in every 2 weeks to 6 months to increase the production of the antibody.

### (7) DNA transgenic animal

The present invention provides a non-human mammal bearing a DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, canine, feline, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, canine, feline, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such asλ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, canine, feline, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

It is possible to obtain homozygous animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening test of an agent for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows an increased symptom of the protein of the present invention liberated, the animal is also expected to serve for screening test of agents preventing/treating the function inactive type inadaptability of the protein of the present invention, for example, agents for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

Other potential applications of two kinds of the DNA transgenic animals of the present invention described above further include:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually cultured only with difficulty, using cells in tissues bearing the DNA cultured by a standard tissue culture technique;
(iv) Screening a drug that enhances the functions of cells using the cells described in (iii) above; and,
(v) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli);*
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. Besides, embryos are preferably collected at the 8-cell stage after culturing until the blastocyte stage and the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities derived from the protein of the present invention, such an animal can be a disease model suspected of inactivated biological activities of the protein of the present invention and thus, offers an effective study to investigate the causes for and therapy for these diseases.

### (8a) Method of screening the compound having a therapeutic/prophylactic effect on diseases caused by deficiency, damages, etc. of the DNA of the present invention

The non-human mammal deficient in expression of the DNA of the present invention can be employed for screening the compound having a therapeutic/prophylactic effect on diseases caused by deficiency, damages, etc. of the DNA of the present invention.

That is, the present invention provides a method of screening the compound having a therapeutic/prophylactic effect on diseases, e.g., cancer, caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

For screening of the compound having a therapeutic/prophylactic effect on cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having the therapeutic/prophylactic effect on the diseases described above.

The compound obtained using the above screening method is a compound selected from the test compounds described above and exhibits a therapeutic/prophylactic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well

The compound obtained by the screening method above may form salts, and may be used in the form of salts with physiologically acceptable acids (e.g., inorganic acids, organic acids, etc.) or bases (e.g., alkali metals, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

The dose of the compound or its salt may vary depending upon target disease, subject to be administered, route of administration, etc. For example, when the compound is orally administered, the compound is administered to the adult patient (as 60 kg body weight) with breast cancer generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient (as 60 kg body weight) with breast cancer in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### (8b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that inhibit the promoter activity to the DNA of the present invention can inhibit the expression of the protein of the present invention and inhibit the functions of the protein. Thus, the compound or its salts are useful as agents for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

In addition, compounds derived from the compound obtained by the screening described above may be used as well.

A pharmaceutical comprising the compound obtained by the above screening method or salts thereof can be manufactured in a manner similar to the method for preparing the pharmaceutical comprising the protein of the present invention described above or salts thereof.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

A dose of the compound or salts thereof may vary depending on target disease, subject to be administered, route for administration, etc.; when the compound that inhibits the promoter activity to the DNA of the present invention is orally administered, the compound is administered to the adult patient (as 60 kg body weight) with breast cancer normally in a daily dose of about 0.1 to about 100 mg, preferably about 1.0 to about 50 mg and more preferably about 1.0 to about 20 mg. In parenteral administration, a single dose of the compound varies depending on subject to be administered, target disease, etc. but when the compound of inhibiting the promoter activity to the DNA of the present invention is administered to the adult patient (as 60 kg body weight) with breast cancer in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification and drawings, where bases, amino acids, etc. are denoted by their codes, they are based on conventional codes in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A: : adenine
- T: : thymine
- G: : guanine
- C: : cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS: :sodium dodecyl sulfate
- Gly: :glycine
- Ala: :alanine
- Val: :valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: :histidine
- Phe: :phenylalanine
- Tyr: :tyrosine
- Trp: :tryptophan
- Pro: :proline
- Asn: :asparagine
- Gln: glutamine
- pGlu: :pyroglutamic acid
- Sec: :selenocysteine

Substituents, protecting groups and reagents frequently used in this specification are presented by the codes described below.
- Me :: methyl group
- Et :: ethyl group
- Bu :: butyl group
- Ph: : phenyl group
- TC: : thiazolidine-4(R)-carboxamido group
- Tos: : p-toluenesulfonyl
- CHO :: formyl
- Bzl: : benzyl
- Cl₂-Bzl :: 2,6-dichlorobenzyl
- Bom :: benzyloxymethyl
- Z: : benzyloxycarbonyl
- Cl-Z: : 2-chlorobenzyloxycarbonyl
- Br-Z: : 2-bromobenzyl oxycarbonyl
- Boc: : t-butoxycarbonyl
- DNP :: dinitrophenol
- Trt: : trityl
- Bum :: t-butoxymethyl
- Fmoc :: N-9-fluorenyl methoxycarbonyl
- HOBt: : 1-hydroxybenztriazole
- HOOBt :: 3,4-dihydro-3-hydroxy-4-oxo-1,2,3-benzotriazine
- HONB: : 1-hydroxy-5-norbornene-2,3-dicarboxyimide
- DCC :: N,N'-dicyclohexylcarbodiimide

The sequence identification numbers in the sequence listing of the specification indicate the following sequences.

### [SEQ ID NO: 1]

This shows the amino acid sequence of Nectin-2α.

### [SEQ ID NO: 2]

This shows the base sequence of DNA encoding Nectin-2α having the amino acid sequence represented by SEQ ID NO: 1.

### [SEQ ID NO: 3]

This shows the amino acid sequence of Nectin-2δ.

### [SEQ ID NO: 4]

This shows the base sequence of DNA encoding Nectin-2δ having the amino acid sequence represented by SEQ ID NO: 3.

### [SEQ ID NO: 5]

This shows the base sequence of the antisense oligonucleotide 1 used in EXAMPLES 1 and 2.

### [SEQ ID NO: 6]

This shows the base sequence of the control oligonucleotide 1 used in EXAMPLES 1 and 2.

### [SEQ ID NO: 7]

This shows the base sequence of the primer 1 used in EXAMPLE 2.

### [SEQ ID NO: 8]

This shows the base sequence of the primer 2 used in EXAMPLE 2.

### [SEQ ID NO: 9]

This shows the base sequence of TaqMan probe 1 used in EXAMPLE 2.

### [SEQ ID NO: 10]

This shows the base sequence of the primer 3 used in EXAMPLE 2.

### [SEQ ID NO: 11]

This shows the base sequence of the primer 4 used in EXAMPLE 2.

### [SEQ ID NO: 12]

This shows the base sequence of TaqMan probe 2 used in EXAMPLE 2.

### [SEQ ID NO: 13]

This shows the base sequence of the primer 5 used in REFERENCE EXAMPLES 1 and 2.

### [SEQ ID NO: 14]

This shows the base sequence of the primer 6 used in REFERENCE EXAMPLE 1.

### [SEQ ID NO: 15]

This shows the base sequence of the primer 7 used in REFERENCE EXAMPLE 2.

### [SEQ ID NO: 16]

This shows the base sequence of the primer 8 used in REFERENCE EXAMPLE 2.

### [SEQ ID NO: 17]

This shows the amino acid sequence of PSEC0110 fis.

### [SEQ ID NO: 18]

This shows the base sequence of DNA encoding PSEC0110 fis having the amino acid sequence represented by SEQ ID NO: 17.

### [SEQ ID NO: 19]

This shows the base sequence of the antisense oligonucleotide 2 used in EXAMPLES 3 and 4.

### [SEQ ID NO: 20]

This shows the base sequence of the control oligonucleotide 2 used in EXAMPLES 3 and 4.

### [SEQ ID NO: 21]

This shows the base sequence of the primer 9 used in EXAMPLE 4.

### [SEQ ID NO: 22]

This shows the base sequence of the primer 10 used in EXAMPLE 4.

### [SEQ ID NO: 23]

This shows the base sequence of the primer 11 used in REFERENCE EXAMPLE 3.

### [SEQ ID NO: 24]

This shows the base sequence of the primer 12 used in REFERENCE EXAMPLE 3.

### [SEQ ID NO: 25]

This shows the amino acid sequence of KIAA0152.

### [SEQ ID NO: 26]

This shows the base sequence of DNA encoding KIAA0152 having the amino acid sequence represented by SEQ ID NO: 25.

### [SEQ ID NO: 27]

This shows the base sequence of the antisense oligonucleotide 3 used in EXAMPLES 5 and 6.

### [SEQ ID NO: 28]

This shows the base sequence of the control oligonucleotide 3 used in EXAMPLES 5 and 6.

### [SEQ ID NO: 29]

This shows the base sequence of the primer 13 used in EXAMPLE 6.

### [SEQ ID NO: 30]

This shows the base sequence or the primer 14 used in EXAMPLE 6.

### [SEQ ID NO: 31]

This shows the base sequence of the primer 30 used in REFERENCE EXAMPLE 6.

### [SEQ ID NO: 32]

This shows the base sequence of the primer 31 used in REFERENCE EXAMPLE 6.

### [SEQ ID NO: 33]

This shows the base sequence of the primer 32 used in REFERENCE EXAMPLE 6.

### [SEQ ID NO: 34]

This shows the base sequence of the primer 17 used in REFERENCE EXAMPLES 4.

### [SEQ ID NO: 35]

This shows the base sequence of the primer 18 used in REFERENCE EXAMPLE 4.

### [SEQ ID NO: 36]

This shows the base sequence of the primer 19 used in REFERENCE EXAMPLE 4.

### [SEQ ID NO: 37]

This shows the base sequence of the primer 20 used in REFERENCE EXAMPLE 4.

### [SEQ ID NO: 38]

This shows the amino acid sequence of DKFZP586L0724.

### [SEQ ID NO: 39]

This shows the base sequence of DNA encoding DKFZP586L0724 having the amino acid sequence represented by SEQ ID NO: 38.

### [SEQ ID NO: 40]

This shows the base sequence of the antisense oligonucleotide 4 used in EXAMPLES 7 and 8.

### [SEQ ID NO: 41]

This shows the base sequence of the control oligonucleotide 4 used in EXAMPLES 7 and 8.

### [SEQ ID NO: 42]

This shows the base sequeuce of the primer 21 used in EXAMPLE 8.

### [SEQ ID NO: 43]

This shows the base sequeuce of the primer 22 used in EXAMPLE 8.

### [SEQ ID NO: 44]

This shows the base sequeuce of the primer 23 used in REFERENCE EXAMPLE 5.

### [SEQ ID NO: 45]

This shows the base sequeuce of the primer 24 used in REFERENCE EXAMPLE 5.

### [SEQ ID NO: 46]

This shows the base sequeuce of the primer 25 used in REFERENCE EXAMPLE 5.

### [SEQ ID NO: 47]

This shows the base sequeuce of the primer 26 used in REFERENCE EXAMPLE 5.

### [SEQ ID NO: 48]

This shows the amino acid sequence of DCBLD1L.

### [SEQ ID NO: 49]

This shows the base sequence of DNA encoding DCBLD1L having the amino acid sequence represented by SEQ ID NO: 48.

### [SEQ ID NO: 50]

This shows the base sequence of the antisence oligonucleotide 5 used in EXAMPLES 9 and 10.

### [SEQ ID NO: 51]

This shows the base sequence of the control oligonucleotide 5 used in EXAMPLES 9 and 10.

### [SEQ ID NO: 52]

This shows the base sequeuce of the primer 27 used in EXAMPLE 10.

### [SEQ ID NO: 53]

This shows the base sequeuce of the primer 28 used in EXAMPLE 10.

### [SEQ ID NO: 54]

This shows the base sequeuce of the primer 29 used in REFERENCE EXAMPLE 6.

### [SEQ ID NO: 55]

This shows the base sequence of siRNA-1 used in EXAMPLES 11, 12 and 13.

### [SEQ ID NO: 56]

This shows the base sequence of siRNA-1 used in EXAMPLES 11, 12 and 13.

### [SEQ ID NO: 57]

This shows the base sequence of siRNA-2 used in EXAMPLES 11, 12 and 13.

### [SEQ ID NO: 58]

This shows the base sequence of siRNA-2 used in EXAMPLES 11, 12 and 13.

### [SEQ ID NO: 59]

This shows the base sequence of siRNA-3 used in EXAMPLES 11, 12 and 13.

### [SEQ ID NO: 60]

This shows the base sequence of siRNA-3 used in EXAMPLES 11, 12 and 13.

### [SEQ ID NO: 61]

This shows the base sequence of siRNA-4 used in EXAMPLES 11, 12 and 13.

### [SEQ ID NO: 62]

This shows the base sequence of siRNA-4 used in EXAMPLES 11, 12 and 13.

### [SEQ ID NO: 63]

This shows the base sequence of siRNA-5 used in EXAMPLES 11, 12 and 13.

### [SEQ ID NO: 64]

This shows the base sequence of siRNA-5 used in EXAMPLES 11, 12 and 13.

### [SEQ ID NO: 65]

This shows the amino acid sequence of the peptide 1 used in EXAMPLE 16.

### [SEQ ID NO: 66]

This shows the amino acid sequence of the peptide 2 used in EXAMPLE 16.

### [SEQ ID NO: 67]

This shows the amino acid sequence of the peptide 3 used in EXAMPLE 16.

### [SEQ ID NO: 68]

This shows the base sequence of the primer 33 used in REFERENCE EXAMPLE 7.

### [SEQ ID NO: 69]

This shows the base sequence of the primer 34 used in REFERENCE EXAMPLE 7.

### [SEQ ID NO: 70]

This shows the amono acid sequence of Nectin-2ED-FLAG protein.

### [SEQ ID NO: 71]

This shows the base sequence of DNA encoding the amino acid sequence of Nectin-2ED-FLAG protein representd by SEQ ID NO: 70.

Hereinafter, the present invention is described in more detail with reference to EXAMPLES and REFERENCE EXAMPLES, but is not deemed to limit the scope of the present invention thereto.

### EXAMPLE 1

### Apoptosis induction in human colon cancer cell line by the addition of antisense oligonucleotide of the Nectin-2α gene and Nectin-2δ gene

Human colon cancer cell line HT-29 purchased from American Type Culture Collection (ATCC) was suspended in McCoy's 5A medium (Invitrogen) [hereinafter sometimes abbreviated as M5 medium] supplemented with 10% fetal calf serum (JRH), and plated on a 96-well flat bottom tissue culture plate (BD Falcon) at a cell density of 10,000 cells/well and then incubated overnight at 37°C in a 5% carbon dioxide gas flow, followed by transfection of the oligonucleotide.

Specifically, after the antisense oligonucleotide sequence (SEQ ID NO: 5) hybridizable to the coding region of Nectin-2α gene or to the intron region of Nectin-2δ gene was designed, the phosphorothioated oligonucleotide was synthesized, purified on HPLC and provided for use (hereinafter merely referred to as the antisense oligonucleotide 1). For control, the oligonucleotide (SEQ ID NO: 6) having a reverse sequence of the base sequence shown by SEQ ID NO: 5 was similarly phosphorothioated, purified on HPLC and provided for use (hereinafter merely referred to as the control oligonucleotide 1). The antisense oligonucleotide 1, 200 ng, or 200 ng of the control oligonucleotide 1 was mixed with 50 µL of Opti-MEM (Invitrogen) together with 0.5 µL of Oligofectamine (Invitrogen) and the mixture was left at room temperature for 20 minutes. The whole volume of the solution mixture above was added to the HT-29 cell culture, which medium had previously been exchanged with 50 µL of Opti-MEM I, the incubation was continued for further 3 hours. Thereafter, the medium was exchanged with M5 medium. After the incubation was continued for further 2 days, the apoptosis induction activity of the oligonucleotide above was determined on Caspase-Glo 3/7 Assay Kit (Promega) in accordance with the protocol attached. As a result, and the antisense oligonucleotide 1 (SEQ ID NO: 5) of the Nectin-2α gene and Nectin-2δ gene showed the apoptosis induction activity of approximately 1.9 times higher than the control oligonucleotide 1 (SEQ ID NO: 6), indicating that there was a statistically significant difference (P<0.05).

### EXAMPLE 2

### Reduction in mRNA expression levels of the Nectin-2α gene and Nectin-2δ gene by the addition of antisense oligonucleotide of the Nectin-2α gene and Nectin-2δ gene

Human colon cancer cell line HT-29 used in EXAMPLE 1 was suspended in M5 medium, and plated on a 24-well flat bottom tissue culture plate (BD Falcon) at a cell density of 60,000 cells/well. The cells were incubated overnight at 37°C in a 5% carbon dioxide gas flow, followed by transfection of the oligonucleotide by a modification of the procedure of EXAMPLE 1, except that the weight or volume of all additives was scaled up to 6 times. Following the transfection, the incubation was continued for 24 hours and the total RNA was then extracted by RNeasy Mini Total RNA Kit (QIAGEN). Using as a template about 400 ng of the total RNA, reverse transcription was carried out on TaqMan Reverse Transcription Reagents (Applied Biosystems) in accordance with the protocol attached thereto. Expression level of the Nectin-2α gene was determined by quantitative PCR; cDNA as a template was used in an amount corresponding to 5 ng when calculated as the total RNA and the reaction solution was made up to 15 µL by adding 7.5 µL of TaqMan Universal PCR Master Mix (Applied Biosystems), 500 nM each of primer 1 (SEQ ID NO: 7) and primer 2 (SEQ ID NO: 8) and 100 nM of FAM-labeled TaqMan probe 1 (SEQ ID NO: 9). The expression level of Nectin-2δ was determined by quantitative PCR, in which cDNA as a template was used in an amount corresponding to 5 ng when calculated as the total RNA and the reaction solution was made up to 15 µL by adding 7.5 µL of TaqMan Universal PCR Master Mix (Applied Biosystems), 500 nM each of primer 3 (SEQ ID NO: 10) and primer 4 (SEQ ID NO: 11) and 100 nM of FAM-labeled TaqMan probe 2 (SEQ ID NO: 12). PCR was carried out by reacting at 50°C for 2 minutes and 95°C for 10 minutes and then repeating 40 times the cycle set to include 95°C for 15 seconds and 60°C for 1 minute. On the other hand, the expression level of a gene for β-actin contained in the same amount of the template cDNA was assayed on TaqMan β-actin Control Reagents (Applied Biosystems), which was used as the internal standard.

Where no oligonucleotide was transfected, the expression levels of the Nectin-2α and Nectin-2δ genes were 0.15% and 0.76% of the expression level of β-actin gene, respectively. In the groups given with the antisense oligonucleotide 1 (SEQ ID NO: 5), expression levels of the Nectin-2α and Nectin-2δ genes were 0.095% and 0.45%, respectively, indicating that a statistically significant (P<0.01) reduction in the expression level was observed when compared to the case where no oligonucleotide was transfected. On the other hand, in the group given with the control oligonucleotide 1 (SEQ ID NO: 6) used as negative control, expression levels of the Nectin-2α and Nectin-2δ genes were 0.18% and 0.76%, respectively, indicating that no statistically significant reduction in the expression level was observed when compared to the case where no oligonucleotide was transfected.

These results reveal that reduction in expression level of the Nectin-2α and Nectin-2δ genes induced the apoptosis of human colon cancer cell line HT-29.

### REFERENCE EXAMPLE 1

### Cloning and base sequencing of cDNA encoding human lung cancer cell-derived protein Nectin-2α

Using human lung cancer cell line A549-derived Marathon-Ready cDNA (CLONTECH) as a template, PCR was carried out by using primer 5 (SEQ ID NO: 13) tagged with a restriction enzyme EcoRI recognition site and primer 6 (SEQ ID NO: 14) tagged with a restriction enzyme EcoRV recognition site. In this reaction, 1 µL of the above cDNA was used as a template and the reaction solution was made up to 20 µL by adding 1 U of PfuTurbo Hotstart DNA Polymerase (STRATAGENE), 1 µM each of primer 5 (SEQ ID NO: 13) and primer 6 (SEQ ID NO: 14), 200 µM of dNTPs and 10 µL of 2 x GC Buffer I (TaKaRa Bio). PCR was carried out by reacting at 94°C for 1 minute and then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. Next, the PCR product was purified using PCR Purification Kit (QIAGEN). The purified product was treated with restriction enzymes EcoRI and EcoRV. pcDNA3.1(+) (Invitrogen) was also treated with restriction enzymes EcoRI and EcoRV. These products were purified on PCR Purification Kit (QIAGEN). The respective DNA fragments were ligated using DNA Ligation Kit ver.2 (TaKaRa Bio) and then transfected to Escherichia coli TOP 10 (Invitrogen), followed by selection in ampicillin-containing LB agar medium. As a result of sequencing of individual clones, animal cell expression vector pcDNA3. (+)-Nectin-2α bearing the cDNA sequence (SEQ ID NO: 2) encoding Nectin-2α protein (SEQ ID NO: 1) was acquired.

### REFERENCE EXAMPLE 2

### Cloning and base sequencing of cDNA encoding human lung cancer cell-derived protein Nectin-2δ

Using human lung cancer cell line A549-derived Marathon-Ready cDNA (CLONTECH) as a template, PCR was carried out by using primer 5 (SEQ ID NO: 13) tagged with a restriction enzyme EcoRI recognition site and primer 7 (SEQ ID NO: 15) tagged with a restriction enzyme EcoRV recognition site. In this reaction, 1 µL of the above cDNA was used as a template and the reaction solution was made up to 20 µL by adding 1 U of PfuTurbo Hotstart DNA Polymerase (STRATAGENE), 1 µM each of primer 5 (SEQ ID NO: 13) and primer 7 (SEQ ID NO: 15), 200 µM of dNTPs and 10 µL of 2 x GC Buffer I (TaKaRa Bio). PCR was carried out by reacting at 94°C for 1 minute and then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. Next, the PCR product was eluted with 50 µL of water using PCR Purification Kit (QIAGEN) and used as a template for PCR. In this reaction, 1 µL of the above PCR product was used as a template and the reaction solution was made up to 20 µL by adding 1 U of PfuTurbo Hotstart DNA Polymerase (STRATAGENE), 1 µM each of primer 5 (SEQ ID NO: 13) and primer 8 (SEQ ID NO: 16) tagged with a restriction enzyme EcoRV recognition site, 200 µM of dNTPs and 10 µL of 2 x GC Buffer I (TaKaRa Bio). PCR was carried out by reacting at 94°C for 1 minute and then repeating 25 times the cycle set to include 94°C for 20 seconds, 60°C for 15 seconds and 72°C for 2 minutes. Next, the PCR product was purified on PCR Purification Kit (QIAGEN). The purified product was treated with restriction enzymes EcoRI and EcoRV. These products were purified on PCR Purification Kit (QIAGEN). The respective DNA fragments were ligated using DNA Ligation Kit ver.2 (TaKaRa Bio) and then transfected to Escherichia coli TOP10 (Invitrogen), followed by selection in ampicillin-containing LB agar medium. As a result of sequencing of individual clones, animal cell expression vector pcDNA3.1(+)-Nectin-2δ bearing the cDNA sequence (SEQ ID NO: 4) encoding Nectin-2δ protein (SEQ ID NO: 3) was acquired.

### EXAMPLE 3

### Apoptosis induction in human colon cancer cell line by the addition of antisense oligonucleotide of the PSEC0110 fis gene

Human colon cancer cell line HT-29 used in EXAMPLE 1 was suspended in M5 medium, plated on a 96-well flat bottom tissue culture plate (BD Falcon) at a cell density of 10,000 cells/well and then incubated overnight at 37°C in a 5% carbon dioxide gas flow, followed by transfection of the oligonucleotide.

Specifically, after the antisense oligonucleotide sequence (SEQ ID NO: 19) hybridizable to a sequence at the 3' untranslated region of PSECO110 fis was designed, the phosphorothioated oligonucleotide was synthesized, purified on HPLC and provided for use (hereinafter merely referred to as the antisense oligonucleotide 2). For control, the oligonucleotide (SEQ ID NO: 20) having a reverse sequence of the base sequence shown by SEQ ID NO: 19 was similarly phosphorothioated, purified on HPLC and provided for use (hereinafter merely referred to as the control oligonucleotide 2). The antisense oligonucleotide 2, 200 ng, or 200 ng of the control oligonucleotide 2 was mixed with 50 µL of Opti-MEM (Invitrogen) together with 0.5 µL of Oligofectamine (Invitrogen) and the mixture was left at room temperature for 20 minutes. The whole volume of the solution mixture above was added to the HT-29 cell culture, which medium had previously been exchanged with 50 µL of Opti-MEM I, the incubation was continued for further 3 hours. Thereafter, the medium was exchanged with M5 medium. After the incubation was continued for further 2 days, the apoptosis induction activity of the oligonucleotide above was determined on Caspase-Glo 3/7 Assay Kit (Promega) in accordance with the protocol attached. As a result, the antisense oligonucleotide 2 (SEQ ID NO: 19) of the PSEC0110 fis gene showed the apoptosis induction activity of approximately 2.9 times higher than the control oligonucleotide 2 (SEQ ID NO: 20), indicating that there was a statistically significant difference (P<0.05).

### EXAMPLE 4

### Reduction in mRNA expression level of the PSEC0110 fis gene by the addition of antisense oligonucleotide of the PSEC0110 fis gene

Human colon cancer cell line HT-29 used in EXAMPLE 1 was suspended in M5 medium, and plated on a 24-well flat bottom tissue culture plate (BD Falcon) at a cell density of 60,000 cells/well. The cells were incubated overnight at 37°C in a 5% carbon dioxide gas flow, followed by transfection of the oligonucleotide by a modification of the procedure of EXAMPLE 3, except that the weight or volume of all additives was scaled up to 6 times. Following the transfection, the incubation was continued for 24 hours and the total RNA was then extracted by RNeasy Mini Total RNA Kit (QIAGEN). Using as a template about 400 ng of the total RNA, reverse transcription was carried out on TaqMan Reverse Transcription Reagents (Applied Biosystems) in accordance with the protocol attached thereto. Expression level of the PSEC0110 fis gene was determined by quantitative PCR, in which cDNA as a template was used in an amount corresponding to 5 ng when calculated as the total RNA and the reaction solution was made up to 15 µL by adding 7.5 µL of SYBR Green PCR Master Mix (Applied Biosystems), 500 nM each of primer 9 (SEQ ID NO: 21) and primer 10 (SEQ ID NO: 22). The expression level was determined by quantitative PCR. PCR was carried out by reacting at 50°C for 2 minutes and 95°C for 10 minutes and then repeating 40 times the cycle set to include 95°C for 15 seconds and 60°C for 1 minute. On the other hand, expression level of a gene for β-actin contained in the same amount of the template cDNA was assayed on TaqMan β-actin Control Reagents (Applied Biosystems), which was used as the internal standard.

Where no oligonucleotide was transfected, expression level of the PSEC0110 fis gene was 0.26% in the gene expression level of β-actin, whereas in the group given with the antisense oligonucleotide 2 (SEQ ID NO: 19), the expression level was 0.17%, indicating that a statistically significant reduction in the expression level was observed (P<0.01). On the other hand, in the group given with the control oligonucleotide 2 (SEQ ID NO: 20) used as negative control, the expression level was 0.23%, indicating that no statistically significant reduction in the expression level was observed when compared to the case where no oligonucleotide was transfected.

These results revealed that reduction in expression level of the PSEC0110 fis gene induced the apoptosis of human colon cancer cell line HT-29.

### REFERENCE EXAMPLE 3

### Cloning and base sequencing of cDNA encoding human lung cancer cell-derived protein PSEC0110 fis

Using human lung cancer cell line A549-derived Marathon-Ready cDNA (CLONTECH) as a template, PCR was carried out by using primer 11 (SEQ ID NO: 23) tagged with a restriction enzyme EcoRI recognition site and primer 12 (SEQ ID NO: 24) tagged with a restriction enzyme XhoI recognition site. In this reaction, 1 µL of the above cDNA was used as a template and the reaction solution was made up to 20 µL by adding 1 U of PfuTurbo Hotstart DNA Polymerase (STRATAGENE), 1 µM each of primer 11 (SEQ ID NO: 23) and primer 12 (SEQ ID NO: 24), 200 µM of dNTPs and 10 µL of 2 x GC Buffer I (TaKaRa Bio). PCR was carried out by reacting at 95°C for 1 minute and then repeating 35 times the cycle set to include 95°C for 20 seconds, 60°C for 15 seconds and 72°C for 3 minutes, followed by reacting at 72°C for 10 minutes. After separation by agarose gel electrophoresis, the DNA fragment corresponding to about 1 kb was recovered and purified using Gel Extraction Kit (QIAGEN). The purified product was subcloned to plasmid vector pCR-BluntII-TOPO (Invitrogen) according to the protocol of Zero Blunt TOPO PCR Cloning Kit (Invitrogen). The clones were transfected to Escherichia coli TOP10 and selected in kanamycin-containing LB agar medium. Sequencing of individual clones gave the plasmid pCR-BluntII-TOPO-PSECO110 fis bearing cDNA sequence (SEQ ID NO: 18) encoding the PSEC0110 fis protein (SEQ ID NO: 17).

Next, pCR-BluntII-TOPO-PSEC0110 fis was treated with restriction enzymes EcoRI and XhoI. pcDNA3.1(+) (Invitrogen) was also treated with restriction enzymes EcoRI and XhoI. These products were purified on PCR Purification Kit (QIAGEN). The respective DNA fragments were ligated using DNA Ligation Kit ver.2 (TaKaRa Bio) and then transfected to Escherichia coli TOP10 (Invitrogen), followed by selection in ampicillin-containing LB agar medium. As a result of sequencing of individual clones, animal cell expression vector pcDNA3.1(+)-PSEC0110 fis bearing the cDNA sequence (SEQ ID NO: 18) encoding PSEC0110 fis protein (SEQ ID NO: 17) was acquired.

### EXAMPLE 5

### Apoptosis induction in colon cancer cell line by the addition of antisense oligonucleotide of the KIAA0152 gene

Human non-small cell lung cancer cell line A549 purchased from ATCC was suspended in F-12 Nutrient Mixture Kaighn's Modified Medium (Invitrogen) [hereinafter sometimes abbreviated as F-12K medium] supplemented with 10% fetal bovine serum (JRH) and plated on a 96-well flat bottom tissue culture plate (BD Falcon) at a cell density of 10,000 cells/well. After incubation at 37°C overnight in a 5% carbon dioxide gas flow, the oligonucleotide was transfected.

Specifically, after the antisense oligonucleotide sequence (SEQ ID NO: 27) hybridizable to a sequence at the 3' untranslated region of KIAA0152 gene was designed, the phosphorothioated oligonucleotide was synthesized, purified on HPLC and provided for use (hereinafter merely referred to as the antisense oligonucleotide 3). For control, the oligonucleotide (SEQ ID NO: 28) having a reverse sequence of the base sequence shown by SEQ ID NO: 27 was similarly phosphorothioated, purified on HPLC and provided for use (hereinafter merely referred to as the control oligonucleotide 3). The antisense oligonucleotide 3, 50 ng, or 50 ng of the control oligonucleotide 3 was mixed with 50 µL of Opti-MEM (Invitrogen) together with 0.8 µL of Lipofectamine 2000 (Invitrogen) and the mixture was left at room temperature for 20 minutes. The whole volume of the solution mixture above was added to the A549 cell culture, which medium had previously been exchanged with 50 µL of Opti-MEM I (Invitrogen), the incubation was continued for further 3 hours. Thereafter, the medium was exchanged with 100 µl of F-12K medium. After the incubation was continued for further 3 days, the apoptosis induction activity of the oligonucleotide above was determined using Cell Death Detection ELISA^{Plus} Kit (Roche Diagnostics) in accordance with the protocol attached. As a result, the antisense oligonucleotide 3 (SEQ ID NO: 27) of the KIAA0152 gene showed the apoptosis induction activity of approximately 1.5 times higher than the control oligonucleotide 3 (SEQ ID NO: 28) used as negative control, indicating that there was a statistically significant difference (P<0.05).

### EXAMPLE 6

### Reduction in mRNA expression level of the KIAA0152 gene by the addition of antisense oligonucleotide of the KIAA0152 gene

Human lung cancer cell line A549 used in EXAMPLE 5 was suspended in F-12K medium, and plated on a 24-well flat bottom tissue culture plate (BD Falcon) at a cell density of 60,000 cells/well. The cells were incubated overnight at 37°C in a 5% carbon dioxide gas flow, followed by transfection of the oligonucleotide by a modification of the procedure of EXAMPLE 5, except that the weight or volume of all additives was scaled up to 6 times. Following the transfection, the incubation was continued for 24 hours and the total RNA was then extracted by RNeasy Mini Total RNA Kit (QIAGEN). Using as a template about 400 ng of the total RNA, reverse transcription was carried out on TaqMan Reverse Transcription Reagents (Applied Biosystems) in accordance with the protocol attached thereto. Expression level of the KIAA0152 gene was determined by quantitative PCR, in which cDNA as a template was used in an amount corresponding to 5 ng when calculated as the total RNA and the reaction solution was made up to 15 µL by adding 7.5 µL of SYBR Green PCR Master Mix (Applied Biosystems), 500 nM each of primer 13 (SEQ ID NO: 29) and primer 14 (SEQ ID NO: 30). The expression level was determined by quantitative PCR. PCR was carried out by reacting at 50°C for 2 minutes and 95°C for 10 minutes and then repeating 40 times the cycle set to include 95°C for 15 seconds and 60°C for 1 minute. On the other hand, the expression level of a gene for β-actin contained in the same amount of the template cDNA was determined and used as the internal standard.

Where no oligonucleotide was transfected, the expression level of KIAA0152 gene was 1.7% of the expression level of β-actin gene, whereas in the group given with the antisense oligonucleotide 3 (SEQ ID NO: 27), the expression level was 1.1%, indicating that a statistically significant reduction in the expression level was observed (P<0.01). On the other hand, in the group given with the control oligonucleotide 3 (SEQ ID NO: 28) used as negative control, the expression level was 1.8%, indicating that no statistically significant reduction in the expression level was observed when compared to the case where no oligonucleotide was transfected.

These results revealed that reduction in expression level of the KIAA0152 gene induced the apoptosis of human lung cancer cell line A549.

### REFERENCE EXAMPLE 4

### Cloning and base sequencing of cDNA encoding human lung cancer cell-derived protein KIAA0152

Using human lung cancer cell line A549-derived Marathon-Ready cDNA (CLONTECH) as a template, PCR was carried out by using primer 17 (SEQ ID NO: 34) tagged with a restriction enzyme EcoRI recognition site and primer 18 (SEQ ID NO: 35) tagged with a restriction enzyme XbaI recognition site. In this reaction, 1 µL of the above cDNA was used as a template and the reaction solution was made up to 20 µL by adding 1.25 U of Platium Pfx DNA Polymerase (Invitrogen), 500 nM each of primer 17 (SEQ ID NO: 34) and primer 18 (SEQ ID NO: 35), 300 µM of dNTPs, 1 mM MgSO₄, 4 µL of 10X PCRx Enhancer System (Invitrogen) and 4 µL of 10X Pfx DNA Polymerase Buffer (Invitrogen). PCR was carried out by reacting at 94°C for 5 minutes and then repeating 35 times the cycle set to include 94°C for 15 seconds, 58°C for 30 seconds and 68°C for 3 minutes. Subsequently, 0.5 U of TaKaRa Ex Taq (TaKaRa Bio) was added to the PCR product. The mixture was reacted at 72°C for 10 minutes. The reaction solution was purified using PCR Purification Kit (QIAGEN). The purified product was subcloned to plasmid vector pCR4-TOPO (Invitrogen) according to the protocol of TOPO TA PCR Cloning Kit (Invitrogen). The clones were transfected to Escherichia coli TOP10F' and selected in kanamycin-containing LB agar medium. Sequencing of individual clones gave plasmid pCR4-TOPO-KIAA0152 bearing cDNA sequence (SEQ ID NO: 26) encoding the KIAA0152 protein (SEQ ID NO: 25).

Next, using pCR4-TOPO-KIAA0152 as a template, PCR was carried out by using primer 19 (SEQ ID NO: 36) tagged with a restriction enzyme EcoRI recognition site and primer 20 (SEQ ID NO: 37) tagged with a restriction enzyme XhoI recognition site. In this reaction, 10 ng of pCR4-TOPO-KIAA0152 was used as a template and the reaction solution was made up to 20 µL by adding 1 U of PfuTurbo Hotstart DNA Polymerase (STRATAGENE), 1 µM each of primer 19 (SEQ ID NO: 36) and primer 20 (SEQ ID NO: 37), 200 µM of dNTPs and 10 µL of 2x GC Buffer I (TaKaRa Bio). PCR was carried out by reacting at 95°C for 1 minute and then repeating 30 times the cycle set to include 95°C for 20 seconds, 60°C for 15 seconds and 72°C for 2 minutes. Next, the PCR product was purified on PCR Purification Kit (QIAGEN). The purified product was then treated with restriction enzymes EcoRI and XhoI. These products were purified on PCR Purification Kit (QIAGEN). The respective DNA fragments were ligated using DNA Ligation Kit ver.2 (TaKaRa Bio) and then transfected to Escherichia coli TOP10 (Invitrogen), followed by selection in ampicillin-containing LB agar medium. As a result of sequencing of individual clones, animal cell expression vector pcDNA3.1(+)-KIAA0152 bearing the cDNA sequence (SEQ ID NO: 26) encoding KIAA0152 protein (SEQ ID NO: 25) was acquired.

### EXAMPLE 7

### Apoptosis induction in lung cancer cell line by the addition of antisense oligonucleotide of the DKFZP586L0724 gene

Human lung cancer cell line A549 used in EXAMPLE 5 was suspended in F-12K medium and plated on a 96-well flat bottomed tissue culture plate (BD Falcon) at a cell density of 10,000 cells/well. After incubation at 37°C overnight in a 5% carbon dioxide gas flow, the oligonucleotide was transfected.

Specifically, after the antisense oligonucleotide sequence (SEQ ID NO: 40) hybridizable to the coding region of DKFZP586L0724 gene was designed, the phosphorothioated oligonucleotide was synthesized, purified on HPLC and provided for use (hereinafter merely referred to as the antisense oligonucleotide 4). For control, the oligonucleotide (SEQ ID NO: 41) having a reverse sequence of the base sequence shown by SEQ ID NO: 40 was similarly phosphorothioated, purified on HPLC and provided for use (hereinafter merely referred to as the control oligonucleotide 4). The antisense oligonucleotide 4, 50 ng, or 50 ng of the control oligonucleotide 4 was mixed with 50 µL of Opti-MEM (Invitrogen) together with 0.8 µL of Lipofectamine 2000 (Invitrogen) and the mixture was left at room temperature for 20 minutes. The whole volume of the solution mixture above was added to the A549 cell culture, which medium had previously been exchanged with 50 µL of Opti-MEM I (Invitrogen), the incubation was continued for further 3 hours. Thereafter, the medium was exchanged with 100 µl of F-12K medium. After the incubation was continued for further 3 days, the apoptosis induction activity of the oligonucleotide above was determined using Caspase-Glo 3/7 Assay Kit (Promega) in accordance with the protocol attached. As a result, the antisense oligonucleotide 4 (SEQ ID NO: 40) of the DKFZP586L0724 gene showed the apoptosis induction activity of approximately 1.5 times higher than the control oligonucleotide 4 (SEQ ID NO: 41) used as negative control, indicating that there was a statistically significant difference (P<0.01).

### EXAMPLE 8

### Reduction in mRNA expression level of the DKFZP586L0724 gene by the addition of antisense oligonucleotide of the DKFZP586L0724 gene

Human lung cancer cell line A549 used in EXAMPLE 5 was suspended in F-12K medium, and plated on a 24-well flat bottom tissue culture plate (BD Falcon) at a cell density of 60,000 cells/well. The cells were incubated overnight at 37°C in a 5% carbon dioxide gas flow. The antisense oligonucleotide 4 (SEQ ID NO: 40) or the control oligonucleotide 4 (SEQ ID NO: 41) was transfected by a modification of the procedure of EXAMPLE 7, except that the weight or volume of all additives was scaled up to 6 times. Following the transfection, the incubation was continued for 20 hours and the total RNA was then extracted by RNeasy Mini Total RNA Kit (QIAGEN). Using as a template 5 µL of the total RNA solution, reverse transcription was carried out on TaqMan Reverse Transcription Reagents (Applied Biosystems) in accordance with the protocol attached thereto. The number of expressed copies of the DKFZP586L0724 gene was calculated by quantitative PCR, in which 2 µL of the cDNA solution thus obtained was used as a template, and the reaction solution was made up to 20 µL by adding 400 nM each of primer 21 (SEQ ID NO: 42) and primer 22 (SEQ ID NO: 43) and 10 µL of SYBR Green PCR Master Mix (Applied Biosystems). PCR was carried out by reacting at 50°C for 2 minutes and 95°C for 10 minutes and then repeating 40 times the cycle set to include 95°C for 15 seconds and 60°C for 1 minute. On the other hand, the expression level of a gene for β-actin contained in the same amount of the template cDNA was determined and used as the internal standard.

Where no oligonucleotide was transfected, expression level of the DKFZP586L0724 gene was 3.2% of the expression level of β-actin gene, whereas in the group given with the antisense oligonucleotide 4 (SEQ ID NO: 40), the expression level was 0.6%. On the other hand, in the group given with the control oligonucleotide 4 (SEQ ID NO: 41), the expression level was 1.4%. When compared to the control oligonucleotide 4 group, a statistically significant reduction in the expression level was observed with the antisense oligonucleotide 4 group (P<0.05). These results revealed that reduction in expression level of the DKFZP586L0724 gene induced the apoptosis of human lung cancer cell line A549.

### REFERENCE EXAMPLE 5

### Cloning and base sequencing of cDNA encoding human lung cancer cell-derived protein DKFZP586L0724

Using human lung cancer cell line A549-derived Marathon-Ready cDNA (CLONTECH) as a template, PCR was carried out by using primer 23 (SEQ ID NO: 44) and primer 24 (SEQ ID NO: 45). In this reaction, 1 µL of the above cDNA was used as a template and the reaction solution was made up to 20 µL by adding 1 U of PfuTurbo Hotstart DNA Polymerase (STRATAGENE), 1 µM each of primer 23 (SEQ ID NO: 44) and primer 24 (SEQ ID NO: 45), 200 µM of dNTPs and 10 µL of 2 x GC Buffer I (TaKaRa Bio). PCR was carried out by reacting at 94°C for 1 minute and then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. Next, using the PCR product as a template, PCR was carried out by using primer 25 (SEQ ID NO: 46) tagged with a restriction enzyme EcoRI recognition site and primer 26 (SEQ ID NO: 47) tagged with a restriction enzyme XhoI recognition site. In this reaction, the PCR product above was diluted in water to 50-fold and 1 µL of the resulting solution was used as a template, and the reaction solution was made up to 20 µL by adding 1 U of PfuTurbo Hotstart DNA Polymerase (STRATAGENE), 1 µM each of primer 25 (SEQ ID NO: 46) and primer 26 (SEQ ID NO: 47), 200 µM of dNTPs and 10 µL of 2x GC Buffer I (TaKaRa Bio). PCR was carried out by reacting at 94°C for 1 minute and then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. After separation by agarose gel electrophoresis, the DNA fragment corresponding to about 2.2 kb was recovered and purified using Gel Extraction Kit (QIAGEN). The purified product was subcloned to plasmid vector pCR-BluntII-TOPO (Invitrogen) according to the protocol of Zero Blunt TOPO PCR Cloning Kit (Invitrogen). The clones were transfected to Escherichia coli TOP 10 (Invitrogen) and selected in kanamycin-containing LB agar medium. Sequencing of individual clones gave the plasmid pCR-BluntII-TOPO-DKFZP586L0724 bearing cDNA sequence (SEQ ID NO: 39) encoding the DKFZP586L0724 protein (SEQ ID NO: 38).

Next, pCR-BluntII-TOPO-DKFZP586L0724 was treated with restriction enzymes BamHI and XhoI. pcDNA3.1 (+) (Invitrogen) was also treated with restriction enzymes BamHI and XhoI. These products were purified on PCR Purification Kit (QIAGEN). The respective DNA fragments were ligated using DNA Ligation Kit ver.2 (TaKaRa Bio) and then transfected to Escherichia coli TOP10 (Invitrogen), followed by selection in ampicillin-containing LB agar medium. As a result of sequencing of individual clones, animal cell expression vector pcDNA3.1(+)-DKFZP586L0724 bearing the cDNA sequence (SEQ ID NO: 39) encoding DKFZP586L0724 protein (SEQ ID NO: 38) was acquired.

### EXAMPLE 9

### Apoptosis induction in lung cancer cell line by the addition of antisense oligonucleotide of the DCBLD1L gene

Lung cancer cell line A549 used in EXAMPLE 5 was suspended in F-12K medium and plated on a 96-well flat bottom tissue culture plate (BD Falcon) at a cell density of 10,000 cells/well. After incubation at 37°C overnight in a 5% carbon dioxide gas flow, the oligonucleotide was transfected.

Specifically, after the antisense oligonucleotide sequence (SEQ ID NO: 50) hybridizable to a sequence at the 3' untranslated region of DCBLD1L gene was designed, the phosphorothioated oligonucleotide was synthesized, purified on HPLC and provided for use (hereinafter merely referred to as the antisense oligonucleotide 5). For control, the oligonucleotide (SEQ ID NO: 51) having a reverse sequence of the base sequence shown by SEQ ID NO: 50 was similarly phosphorothioated, purified on HPLC and provided for use (hereinafter merely referred to as the control oligonucleotide 5). The antisense oligonucleotide 5, 50 ng, or 50 ng of the control oligonucleotide 5 was mixed with 50 µL of Opti-MEM (Invitrogen) together with 0.8 µL of Lipofectamine 2000 (Invitrogen) and the mixture was left at room temperature for 20 minutes. The whole volume of the solution mixture above was added to the A549 cell culture, which medium had previously been exchanged with 50 µL of Opti-MEM I (Invitrogen), the incubation was continued for further 3 hours. Thereafter, the medium was exchanged with 100 µl of F-12K medium. After the incubation was continued for further 3 days, the apoptosis induction activity of the oligonucleotide above was determined using Caspase-Glo 3/7 Assay Kit (Promega) in accordance with the protocol attached. As a result, the antisense oligonucleotide 5 (SEQ ID NO: 50) of the DCBLD1L gene showed the apoptosis induction activity of approximately 1.3 times higher than the control oligonucleotide 5 (SEQ ID NO: 51) used as negative control, indicating that there was a statistically significant difference (P<0.01).

### EXAMPLE 10

### Reduction in mRNA expression level of the DCBLD1L gene by the addition of antisense oligonucleotide of the DCBLD1L gene

Human lung cancer cell line A549 used in EXAMPLE 5 was suspended in F-12K medium, and plated on a 24-well flat bottom tissue culture plate (BD Falcon) at a cell density of 60,000 cells/well. The cells were incubated overnight at 37°C in a 5% carbon dioxide gas flow. The antisense oligonucleotide 5 (SEQ ID NO: 50) or the control oligonucleotide 5 (SEQ ID NO: 51) was transfected by a modification of the procedure of EXAMPLE 9, except that the weight or volume of all additives was scaled up to 6 times. Following the transfection, the incubation was continued for 20 hours and the total RNA was then extracted by RNeasy Mini Total RNA Kit (QIAGEN). Using as a template 5 µL of the total RNA solution, reverse transcription was carried out on TaqMan Reverse Transcription Reagents (Applied Biosystems) in accordance with the protocol attached thereto. The copy number of the DCBLD1L gene expressed was calculated by quantitative PCR, in which 2 µL of the cDNA solution thus obtained was used as a template, and the reaction solution was made up to 20 µL by adding 400 nM each of primer 27 (SEQ ID NO: 52) and primer 28 (SEQ ID NO: 53) and 10 µL of SYBR Green PCR Master Mix (Applied Biosystems). PCR was carried out by reacting at 50°C for 2 minutes and 95°C for 10 minutes and then repeating 40 times the cycle set to include 95°C for 15 seconds and 60°C for 1 minute. On the other hand, the expression level of a gene for β-actin contained in the same amount of the template cDNA was determined and used as the internal standard.

Where no oligonucleotide was transfected, expression level of the DCBLD1L gene was 2.2% of the expression level of β-actin gene, whereas in the group given with the antisense oligonucleotide 5 (SEQ ID NO: 50), the expression level was 0.3%. On the other hand, in the group given with the control oligonucleotide 5 (SEQ ID NO: 51), the expression level was 1.5%. When compared to the control oligonucleotide 5 group, a statistically significant reduction in the expression level was observed with the antisense oligonucleotide 5 group (P<0.01). These results revealed that reduction in expression level of the DCBLD1L gene induced the apoptosis of human lung cancer cell line A549.

### REFERENCE EXAMPLE 6

### Cloning and base sequencing of cDNA encoding human lung cancer cell-derived protein DCBLD1L

Using human lung cancer cell line A549-derived Marathon-Ready cDNA (CLONTECH) as a template, PCR was carried out by using primer 29 (SEQ ID NO: 54) and primer 30 (SEQ ID NO: 31). In this reaction, 1 µL of the above cDNA was used as a template and the reaction solution was made up to 20 µL by adding 1 U of PfuTurbo Hotstart DNA Polymerase (STRATAGENE), 1 µM each of primer 29 (SEQ ID NO: 54) and primer 30 (SEQ ID NO: 31), 200 µM of dNTPs and 10 µL of 2x GC Buffer I (TaKaRa Bio). PCR was carried out by reacting at 95°C for 1 minute and then repeating 40 times the cycle set to include 96°C for 20 seconds, 63°C for 15 seconds and 72°C for 3 minutes. After separation by agarose gel electrophoresis, the DNA fragment corresponding to about 2.3 kb was recovered and purified using Gel Extraction Kit (QIAGEN). The purified product was subcloned to plasmid vector pCR-BluntII-TOPO (Invitrogen) according to the protocol of Zero Blunt TOPO PCR Cloning Kit (Invitrogen). The clones were transfected to Escherichia coli TOP10 (Invitrogen) and selected in kanamycin-containing LB agar medium. Sequencing of individual clones gave the plasmid pCR-BluntII-TOPO-DCBLD1L bearing cDNA sequence (SEQ ID NO: 49) encoding the DCBLD1L protein (SEQ ID NO: 48).

Next, using pCR-BluntII-TOPO-DCBLD1L as a template, PCR was performed by using primer 31 (SEQ ID NO: 32) and primer 32 (SEQ ID NO: 33). In this reaction, 10 ng of pCR-BluntII-TOPO-DCBLD1L above was used as a template and the reaction solution was made up to 20 µL by adding 1U of PfuTurbo Hotstart DNA Polymerase (STRATAGENE), 1 µM each of primer 31 (SEQ ID NO: 32) tagged with a restriction enzyme EcoRI recognition site and primer 32 (SEQ ID NO: 33) tagged with a restriction enzyme XhoI recognition site, 200 µM of dNTPs and 10 µL of 2xGC Buffer I (TaKaRa Bio). PCR was performed by reacting at 94°C for 1 minute and then repeating 25 times the cycle set to include 94°C for 20 seconds, 60°C for 15 seconds and 72°C for 2 minutes. The PCR product was purified and treated with restriction enzymes EcoRI and XhoI. pcDNA3.1(+) (Invitrogen) was also treated with restriction enzymes EcoRI and XhoI. The respective DNA fragments were separated by agarose gel electrophoresis and purified using Gel Extraction Kit (QIAGEN). The respective DNA fragments were ligated using DNA Ligation Kit ver.2 (TaKaRa Bio) and then transfected to Escherichia coli TOP10 (Invitrogen), followed by selection in ampicillin-containing LB agar medium. As a result of sequencing of individual clones, animal cell expression vector pcDNA3.1(+)-DCBLD1L bearing the cDNA sequence (SEQ ID NO: 49) encoding DCBLD1L protein (SEQ ID NO: 48) was acquired.

### EXAMPLE 11

### Cell growth suppression of human colon cell line by the addition of siRNA to mRNA for Nectin-2 gene

The siRNAs to mRNA of the Nectin-2α gene or the Nectin-2δ gene (hereinafter collectively referred to as the Nectin-2 gene) were prepared by equally mixing five siRNAs (siRNA-1, siRNA-2, siRNA-3, siRNA-4 and siRNA-5) to mRNA of the Nectin-2α gene or the Nectin-2δ gene (hereinafter the siRNAs obtained by mixing siRNA-1, siRNA-2, siRNA-3, siRNA-4 and siRNA-5 are referred to as the Nectin-2-siRNA). siRNA-1, siRNA-2, siRNA-3, siRNA-4 and siRNA-5, which are the siRNAs to mRNA of the Nectin-2α gene or the Nectin-2δ gene, were prepared by hybridizing two RNA fragments, respectively (siRNA-1 was prepared by hybridizing RNA having the base sequence represented by SEQ ID NO: 55 to RNA having the base sequence represented by SEQ ID NO: 56, siRNA-2 by hybridizing RNA having the base sequence represented by SEQ ID NO: 57 to RNA having the base sequence represented by SEQ ID NO: 58, siRNA-3 by hybridizing RNA having the base sequence represented by SEQ ID NO: 59 to RNA having the base sequence represented by SEQ ID NO: 60, siRNA-4 by hybridizing RNA having the base sequence represented by SEQ ID NO:61 to RNA having the base sequence represented by SEQ ID NO: 62, and siRNA-5 by hybridizing RNA having the base sequence represented by SEQ ID NO: 63 to RNA having the base sequence represented by SEQ ID NO: 64). For negative control, non-specific Control IX (hereinafter briefly referred to as non-silencing dsRNA) purchased from Dharmacon was used.

Specifically, human colon cancer cell line HT-29 purchased from American Type Culture Collection (ATCC) was suspended in M5A medium supplemented with 10% fetal bovine serum (JRH) and plated on a 10 cm tissue culture Petri dish (BD Falcon) at a cell density of 500,000 cells/well. After incubation overnight at 37°C in a 5% carbon dioxide gas flow, HT-29 cells were recovered using trypsin/EDTA. One million of the recovered HT-29 cells were suspended in 100 µl of solution V attached to Cell Line Nucleofector Kit V (Amaxa), which solution contained 150 pmol of Nectin-2-siRNA or 150 pmol of non-silencing dsRNA, and transfected using Nucleofector program T-20. After incubation at 37°C for 24 hours in a 5% carbon dioxide gas flow, the cells were plated on a 96-well flat bottomed tissue culture plate at a cell density of 3,000 cells/well and the incubation was continued for 5 days. Subsequently, after the medium was removed, the plate was settled at -80°C for 5 minutes and allowed to stand at room temperature for 5 minutes. Next, 100 µL of an aqueous solution containing 1% PicoGreen (Molecular Probes) and 1% IGEPAL-CA630 (ICN) was added to each well, which was allowed to stand for 20 minutes. Then, the fluorescence intensity was measured at 485 nm excitation wavelength and 535 nm emission wavelength to determine the DNA level in the cells. As a result, the fluorescence intensity decreased by about 38% in the Nectin-2-siRNA group, when compared to the non-silencing dsRNA group, indicating that there was a statistically significant difference (P<0.001). This reveals the growth suppression of Nectin-2-siRNA against HT-29 cells.

### EXAMPLE 12

### Change in cell cycle of HT-29 cells by the addition of Nectin-2-siRNA

Human colon cancer cell line HT-29 used in EXAMPLE 11 was suspended in M5A medium and plated on a 10 cm tissue culture Petri dish at a cell density of 500,000 cells/well. After incubation overnight at 37°C in a 5% carbon dioxide gas flow, Nectin-2-siRNA or non-silencing dsRNA as negative control was transfected by a modification of the procedure of EXAMPLE 11. The incubation was continued for 24 hours. HT-29 cells were then recovered, plated on a 6-well flat bottom tissue culture plate (BD Falcon) at a cell density of 200,000 cells/well and incubated at 37°C in a 5% carbon dioxide gas flow. Following the incubation for 5 days, cell cycle analysis was performed on FACScan (Becton Dickinson) using CycleTEST Plus DNA Reagent Kit (Becton Dickinson). As a result, the ratio of cells in the G₀/G₁ phase increased by about 13% in the Nectin-2-siRNA group, as compared to the non-silencing dsRNA group used as negative control. Furthermore, the ratio of cells in the S-phase decreased by about 11 % in the Nectin-2-siRNA group, as compared to the non-silencing dsRNA group. The results indicate that the change in cell cycle of human colon cancer cell line HT-29 was induced by Nectin-2-siRNA.

### EXAMPLE 13

### Reduction in mRNA expression level of the Nectin-2 gene by the addition of Nectin-2-siRNA

Human colon cancer cell line HT-29 used in EXAMPLE 11 was suspended in M5A medium and plated on a 10 cm tissue culture Petri dish at a cell density of 500,000 cells/well. After incubation overnight at 37°C in a 5% carbon dioxide gas flow, Nectin-2-siRNA or non-silencing dsRNA as negative control was transfected by a modification of the procedure of EXAMPLE 11. Following the transfection, the incubation was continued for 24 hours and the total RNA was extracted by RNeasy Mini Total RNA Kit (QIAGEN). Using as a template about 100 ng of the total RNA, reverse transcription was performed on TaqMan Reverse Transcription Reagents (Applied Biosystems) in accordance with the protocol attached thereto. The expression level of mRNA of the Nectin-2α gene was determined by quantitative PCR, in which cDNA as a template was used in an amount corresponding to 10 ng when calculated as the total RNA and the reaction solution was made up to 10 µL by adding 5 µL of TaqMan Universal PCR Master Mix (Applied Biosystems), 500 nM each of primer 1 (SEQ ID NO: 7) and primer 2 (SEQ ID NO: 8) and 100 nM of FAM-labeled TaqMan probe 1 (SEQ ID NO: 9). On the other hand, the expression level of mRNA of the Nectin-2δ gene was determined by quantitative PCR, in which cDNA as a template was used in an amount corresponding to 10 ng when calculated as the total RNA and the reaction solution was made up to 10 µL by adding 5 µL of TaqMan Universal PCR Master Mix (Applied Biosystems), 500 nM each of primer 3 (SEQ ID NO: 10) and primer 4 (SEQ ID NO: 11) and 100 nM of FAM-labeled TaqMan probe 2 (SEQ ID NO: 12). PCR was carried out by reacting at 50°C for 2 minutes and 95°C for 10 minutes and then repeating 40 times the cycle set to include 95°C for 15 seconds and 60°C for 1 minute. On the other hand, the expression level of mRNA for β-actin contained in the same amount of the template cDNA was determined and used as the internal standard.

The expression levels of mRNA of Nectin-2α and Nectin-2δ decreased by 69% and 73%, respectively, in the Nectin-2-siRNA group, when compared to the non-silencing dsRNA group used as negative control, indicating that there was a statistically significant difference (P<0.001). These results indicate that reduction in expression levels of mRNA of Nectin-2α and Nectin-2δ was induced by the addition of Nectin-2-siRNA.

### EXAMPLE 14

### Study of increased expression of mRNA for the Nectin-2 gene in human cancer tissues

A study was conducted by quantitative PCR to see if mRNA expression for the Nectin-2 gene was increased in cancer tissues. For quantification of the expression level, cDNA CeHAT-SD Breast Tumor 1 (Cosmobio), cDNA CeHAT-SD Breast Tumor 2 (Cosmobio), Human Colon Matched cDNA Pair Panel (CLONTECH), Human Lung Matched cDNA Pair Panel (CLONTECH) and Human Ovary Matched cDNA Pair Panel (CLONTECH) were used. The expression level of mRNA for the Nectin-2α gene was determined as follows: 1 µL of cDNA was used as a template and the reaction solution was made up to 15 µL by adding 7.5 µL of TaqMan Universal PCR Master Mix (Applied Biosystems), 500 nM each of primer 1 (SEQ ID NO: 7) and primer 2 (SEQ ID NO: 8) and 100 nM of FAM-labeled TaqMan probe 1 (SEQ ID NO: 9). Expression level of mRNA for the Nectin-2δ gene was determined as follows: 1 µL of cDNA was used as a template and the reaction solution was made up to 15 µL by adding 7.5 µL of TaqMan Universal PCR Master Mix (Applied Biosystems), 500 nM each of primer 3 (SEQ ID NO: 10) and primer 4 (SEQ ID NO: 11) and 100 nM of FAM-labeled TaqMan probe 2 (SEQ ID NO: 12), except that the amount of templates for cDNA CeHAT-SD Breast Tumor 1 (Cosmobio) and cDNA CeHAT-SD Breast Tumor 2 (Cosmobio) was 0.2 µL. PCR was performed by reacting at 50°C for 2 minutes and 95°C for 10 minutes and then repeating 40 times the cycle set to include 95°C for 15 seconds and 60°C for 1 minute. On the other hand, the expression level of mRNA for β-actin contained in the same amount of the template cDNA was determined and used as the internal standard. As a result, expression level of mRNA for the Nectin-2α gene in cancer tissues increased with 3 donors included in cDNA CeHAT-SD Breast Tumor 1 (Cosmobio) by 1.1 times, 10 times and 4.3 times, respectively, and increased with 3 donors included in cDNA CeHAT-SD Breast Tumor 2 (Cosmobio) by 12 times, 3.5 times and 21 times, respectively, when compared to the expression level in normal tissues. Similarly, the increased expression level was confirmed with 5 donors included in Human Colon Matched cDNA Pair Panel (CLONTECH) to be 4.8 times, 3.2 times, 2.6 times, 1.9 times and 1.8 times, respectively; with 5 donors included in Human Lung Matched cDNA Pair Panel (CLONTECH) to be 11 times, 3.7 times, 4.1 times, 3.2 times and 1.3 times, respectively; and, with 4 out of 5 donors included in Human Ovary Matched cDNA Pair Panel (CLONTECH) to be 1.3 times, 1.8 times, 2.6 times and 2.4 times, respectively. An increased expression level of mRNA for the Nectin-2δ gene was noted with 2 out of 3 donors included in cDNA CeHAT-SD Breast Tumor 1 (Cosmobio), which was 1.1 times and 5.3 times, respectively; and, with 2 out of 3 donors included in cDNA CeHAT-SD Breast Tumor 2 (Cosmobio), which was 2.0 times and 2.5 times, respectively. Similarly, the increased expression level was confirmed with 3 out of 5 donors included in Human Colon Matched cDNA Pair Panel (CLONTECH) to be 1.3 times, 1.8 times and 1.5 times, respectively; with 4 out of 5 donors included in Human Lung Matched cDNA Pair Panel (CLONTECH) to be 4.8 times, 3.7 times, 1.1 times and 1.3 times, respectively; and with 4 out of 5 donors included in Human Ovary Matched cDNA Pair Panel (CLONTECH) to be 4.2 times, 2.1 times, 2.4 times and 4.2 times, respectively. From these results, overexpression of mRNA for the Nectin-2α gene and Nectin-2δ gene in cancer tissues was confirmed.

### EXAMPLE 15

### Quantification of mRNA for the Nectin-2 gene in human cancer cell lines

Osteosarcoma cell line Saos-2; brain tumor cell lines SK-N-MC, SK-N-AS, SK-N-BE, SK-N-DZ, SK-N-FI, SK-N-SH, D341 Med, Daoy, DBTRG-05MG, U-118 MG, U-87 MG, CCF-STTG1 and SW 1088; breast cancer cell lines HCC1937, ZR-75-1, AU565, MCF-7, MDA-MB-231, SKBR-3, BT474, MDA-MB-435s, MDA-MB-436, MDA-MB-468, MDA-MB-175VII and T-47D; colon cancer cell lines Caco-2, COLO 201, COLO 205, COLO 320DM, DLD-1, HCT-15, HCT-8, HT-29, LoVo, LS180, LS123, LS174T, NCI-H548, NCI-SNU-C1, SK-CO-1, SW 403, SW 48, SW 480, SW 620, SW 837, SW 948, HCT 116 and WiDr; non-small cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1435, NCI-H1581, NCI-H1651, NCI-H1703, NCI-H1793, NCI-H2073, NCI-H2085, NCI-H2106, NCI-H2228, NCI-H2342, NCI-H2347, SK-LU-1, NCI-H2122, SK-MES-1 and NCI-H292; small cell lung cancer cell lines NCI-H187, NCI-H378, NCI-H526, NCI-H889, NCI-H1417, NCI-H1672, NCI-H1836, NCI-H1963, NCI-H2227, NCI-N417 and SHP-77; ovary cancer cell lines ES-2, Caov-3, MDAH2774, NIH:OVCAR3, OV-90, SK-OV-3, TOV-112D and TOV-21 G; prostate cancer cell lines DU 145 and LNCaP; human retinoblastoma cell lines WERI-Rb-1 and Y79, testicular cancer cell line Cates-1B (all purchased from ATCC); colon cancer cell line COCM1; non-small cell lung cancer cell line VMRC-LCD and prostate cancer cell line PC3 (all purchased from Japanese Collection of Research Bioresources (JCRB)) were incubated, respectively, in accordance with the incubation protocol recommended by ATCC or JCRB. The total RNA was prepared by using RNeasy Mini Total RNA Kit (QIAGEN). Using this total RNA as a template, reverse transcription was performed to prepare cDNA. Quantitative PCR was carried out to quantify the expression level of mRNA for the Nectin-2 gene. The expression level of mRNA for the Nectin-2 gene was quantified by the procedure described in EXAMPLE 2, using as a template the cDNA obtained from 5 ng of the total RNA above. On the other hand, the expression level of a gene for β-actin contained in the same amount of the template cDNA was determined and used as the internal standard.

Relative expression levels obtained by standardization of the expression level of mRNA for the Nectin-2α gene or the Nectin-2δ gene with the expression level of mRNA for the β-actin gene are shown in [TABLE 1]. The results reveal that expression level of mRNA for the Nectin-2α gene was 1% or higher in 2 strains of the cancer cell lines and for the Nectin-2δ gene in 12 strains of the cancer cell lines, respectively, when compared to the expression level of β-actin.

**[TABLE 1]**

| Cell name | Necti n-2α | Necti n-2δ | Cell name | Necti n-2α | Nectin-2δ | Cell name | Nectin-2α | Necti n-2δ |
|---|---|---|---|---|---|---|---|---|
| Saos-2 | 0.04 | 0.10 | HCT-8 | 0.36 | 1.44 | NCI-H1155 | 0.06 | 0.06 |
| CCF-STTG 1 | 0.19 | 0.39 | HT-29 | 0.35 | 1.93 | NCI-H1299 | 0.57 | 0.82 |
| SW 1088 | 0.17 | 0.11 | LoVo | 0.16 | 0.46 | NCI-H1581 | 0.19 | 0.61 |
| DBTRG-05 MG | 0.08 | 0.18 | LS 180 | 0.17 | 0.36 | NCI-H2106 | 0.05 | 0.13 |
| U-118 MG | 0.26 | 0.08 | LS123 | 0.29 | 0.90 | NCI-H187 | 0.00 | 0.01 |
| U-87 MG | 0.13 | 0.11 | LS 174T | 0.08 | 0.44 | NCI-H378 | 0.06 | 0.11 |
| D341 Med | 0.11 | 0.09 | NCI-H548 | 1.11 | 2.07 | NCI-H526 | 0.26 | 0.41 |
| Daoy | 0.13 | 0.11 | NCI-SNU-C1 | 0.19 | 0.30 | NCI-H889 | 0.07 | 0.19 |
| SK-N-AS | 0.06 | 0.04 | SK-CO-1 | 0.57 | 1.35 | NCI-H1417 | 0.08 | 0.20 |
| SK-N-BE | 0.03 | 0.04 | SW 403 | 0.12 | 0.49 | NCI-H1672 | 0.07 | 0.51 |
| SK-N-DZ | 0.03 | 0.03 | SW 48 | 0.21 | 0.22 | NCI-H1836 | 0.12 | 0.27 |
| SK-N-FI | 0.12 | 0.17 | SW 480 | 0.14 | 0.26 | NCI-H1963 | 0.04 | 0.05 |
| SK-N-SH | 0.09 | 0.19 | SW 620 | 0.10 | 0.38 | NCI-H2227 | 0.12 | 0.36 |
| SK-N-MC | 0.10 | 0.09 | SW 837 | 0.36 | 1.27 | NCI-N417 | 0.00 | 0.00 |
| AU565 | 0.05 | 0.13 | SW 948 | 0.56 | 1.19 | SHP-77 | 0.10 | 0.33 |
| MCF-7 | 0.08 | 0.68 | WiDr | 0.21 | 1.92 | NCI-H226 | 0.04 | 0.26 |
| MDA-MB-231 | 0.08 | 0.11 | A549 | 0.24 | 0.25 | NCI-H1703 | 0.30 | 0.48 |
| SK-BR-3 | 0.31 | 0.65 | NCI-H23 | 0.15 | 0.24 | NCI-H2122 | 0.02 | 0.17 |
| BT474 | 0.19 | 0.58 | NCI-H358 | 0.12 | 0.46 | SK-MES-1 | 0.04 | 0.12 |
| HCC1937 | 0.15 | 0.29 | NCI-H522 | 0.20 | 0.18 | NCI-H292 | 0.00 | 1.03 |
| MDA-MB-435s | 0.08 | 0.12 | NCI-H1395 | 0.16 | 0.39 | Caov-3 | 0.08 | 0.30 |
| ZR-75-1 | 0.63 | 1.57 | NCI-H1435 | 0.40 | 0.46 | MDAH277 4 | 0.12 | 0.17 |
| MDA-MB-436 | 0.08 | 0.15 | NCI-H1651 | 0.07 | 0.21 | NIH:OVCA R3 | 0.17 | 0.43 |
| MDA-MB-468 | 0.04 | 0.26 | NCI-H1793 | 0.13 | 0.25 | OV-90 | 1.09 | 5.06 |
| MDA-MB-175VII | 0.03 | 0.12 | NCI-H2073 | 0.15 | 0.34 | SK-OV-3 | 0.32 | 0.72 |
| T-47D | 0.08 | 0.40 | NCI-H2085 | 0.20 | 0.34 | TOV-112D | 0.46 | 0.45 |
| COCM1 | 0.22 | 0.77 | NCI-H2228 | 0.34 | 0.44 | TOV-21G | 0.24 | 0.25 |
| Caco-2 | 0.37 | 0.99 | NCI-H2342 | 0.64 | 2.45 | ES-2 | 0.20 | 0.28 |
| COLO 201 | 0.16 | 0.40 | NCI-H2347 | 0.05 | 0.12 | DU 145 | 0.14 | 0.60 |
| COLO 205 | 0.23 | 0.63 | SK-LU-1 | 0.04 | 0.10 | LNCaP | 0.29 | 0.60 |
| COLO 320DM | 0.15 | 0.24 | VMRC-LCD | 0.12 | 0.10 | PC3 | 0.14 | 0.24 |
| DLD-1 | 0.35 | 1.26 | NCI-H460 | 0.12 | 0.15 | Y79 | 0.11 | 0.19 |
| HCT 116 | 0.40 | 0.71 | NCI-H661 | 0.13 | 0.44 | WERI-Rb-1 | 0.25 | 0.54 |
| HCT-15 | 0.43 | 0.76 | NCI-H810 | 0.09 | 0.20 | Cates-1B | 0.16 | 0.18 |

### EXAMPLE 16

### Production of rabbit anti-Nectin-2 polyclonal antibody using peptide antigen

Based on the amino acid sequences of Nectin-2α protein (SEQ ID NO: 1) and Nectin-2δ protein (SEQ ID NO: 3), the following 3 peptides (peptides 1 - 3) consisting of 15 amino acids were synthesized.

### Amino acid sequence of peptide 1

[Cys-Lys-Met-Gly-Pro-Ser-Phe-Pro-Ser-Pro-Lys-Pro-Gly-Ser-Glu (SEQ ID NO: 65)]

Peptide 1 is a sequence wherein Cys is added to the 88-101 amino acid sequence of Nectin-2α protein (SEQ ID NO: 1) and Nectin-2δ protein (SEQ ID NO: 3) at its N terminus.

### Amino acid sequence of peptide 2

[Arg-Glu-Thr-Pro-Arg-Ala-Ser-Pro-Arg-Asp-Val-Gly-Pro-Leu-Cys (SEQ ID NO: 66)]

Peptide 2 is a sequence wherein Cys is added to the 347-360 amino acid sequence of Nectin-2α protein (SEQ ID NO: 1) at its C terminus.

### Amino acid sequence of peptide 3

[Cys-Thr-Leu-Gly-Ala-Ser-Glu-His-Ser-Pro-Leu-Lys-Thr-Pro-Tyr (SEQ ID NO: 67)]

Peptide 3 is a sequence wherein Cys is added to the 426-439 amino acid sequence of Nectin-2δ protein (SEQ ID NO: 3) at its N terminus.

Keyhole limpet hemocyanin (KLH) as a carrier protein was coupled to each of peptide 1, peptide 2 and peptide 3, which was used as an antigen. Male rabbit KBL:JW (11 weeks old, Oriental Yeast) was used as an immunized animal. Freund's complete adjuvant (Difco) suspension was used for primary immunization and Freund's incomplete adjuvant (Difco Laboratories) suspension was used for the second and subsequent immunization. The immunization was effected by subcutaneous injection at the back and 0.5 mg of each antigen was used per immunization. After the primary immunization, booster was repeated 3 times every 14 days. On day 52 after the primary immunization, blood was collected through the carotid artery under anesthesia to give the sera of 70 ml, 66 ml and 72 ml, respectively. The sera thus obtained were concentrated with ammonium sulfate salting out and purified through a protein A-affinity column (Amersham-Bioscience) to give the purified IgG from the rabbit immunized with peptides 1, 2 and 3. The purified IgG thus obtained was used to purify on a peptide immobilized column. For the immobilization, Cys of each peptide was utilized and the peptide was coupled to a Sepharose column (Amersham-Bioscience) using borate buffer. For elution from the column, 8M urea-containing PBS was used. The eluate was dialyzed to PBS to remove urea, which was followed by ultraconcentration and sterilization by filtering. Thus, the purified anti-Nectin-2 polyclonal antibodies AS-2704, AS-2705 and AS-2706 to peptides 1, 2 and 3 were acquired.

### REFERENCE EXAMPLE 7

### Construction of animal cell expression vector for recombinant Nectin-2 extracellular domain protein

Using as a template pcDNA3.1(+)-Nectin-2δ prepared in REFERENCE EXAMPLE 2, PCR was carried out by using primer 33 (SEQ ID NO: 68) tagged with a restriction enzyme EcoRI recognition site and primer 34 (SEQ ID NO: 69) tagged with a restriction enzyme XhoI recognition site. In this reaction, 10 ng of pcDNA3.1(+)-Nectin-2δ was used as a template and the reaction solution was made up to 50 µL by adding 2.5 U of PfuUltra Hotstart DNA Polymerase (STRATAGENE), 0.2 µM each of primer 33 (SEQ ID NO: 68) and primer 34 (SEQ ID NO: 69), 200 µM of dNTPs and 5 µL of 10x Pfu Ultra Buffer (TaKaRa Bio). PCR was carried out by reacting at 95°C for 2 minutes and then repeating 30 times the cycle set to include 95°C for 30 seconds, 60°C for 30 seconds and 72°C for 1 minute and 15 seconds, followed by reacting at 72°C for 10 minutes. Next, the PCR product was purified on PCR Purification Kit (QIAGEN). The purified product was then treated with restriction enzymes EcoRI and XhoI. Similarly, pCMV-Tag4 (STRATAGENE) was treated with restriction enzymes EcoRI and XhoI. Each DNA fragment was purified using Wizard SV Gel and PCR Clean-Up System (Promega) and subjected to ligation using Ligation High (TOYOBO). The plasmid obtained was transfected to Escherichia coli TOP10 (Invitrogen) and selected in kanamycin-containing LB agar medium. As a result of sequencing of individual clones, animal cell expression vector pCMV-Tag4-Nectin-2ED-FLAG bearing the cDNA sequence (SEQ ID NO: 71) encoding Nectin-2ED-FLAG protein (SEQ ID NO: 70) with a FLAG tag at the C terminus in the extracellular domain (1-361 amino acid sequence of Nectin-2δ represented by SEQ ID NO: 3) of Nectin-2δ protein was acquired.

### EXAMPLE 17

### Preparation of recombinant Nectin-2ED-FLAG protein

Nectin-2ED-FLAG protein encoded by pCMV-Tag4-Nectin-2ED-FLAG prepared in REFERENCE EXAMPLE 7 was acquired using FreeStyle293 Expression System (Invitrogen). Specifically, pCMV-Tag4-Nectin-2ED-FLAG was transfected to 293F cell line using 293 Fectin (Invitrogen), followed by rotation culture at 37°C for 3 days in a 5% carbon dioxide gas flow. The cell suspension was centrifuged and the resulting culture supernatant was filtrated through a 0.45 µm filter and the filtrate was passed through an anti-FLAG antibody column (Sigma) equilibrated with PBS. After washing the column with PBS, elution was performed with PBS containing 0.1 mg/mL of the FLAG peptide. After the eluted fraction of the Nectin-2ED-FLAG protein was concentrated by ultrafiltration, the FLAG peptide was removed by desalting gel filtration column PD-10 (Amersham Biosciences) equilibrated with PBS and concentrated again to acquire recombinant Nectin-2ED-FLAG protein.

### EXAMPLE 18

### Preparation of rabbit anti-Nectin-2 polyclonal antibody using Nectin-2ED-FLAG protein

Rabbit anti-Nectin-2 polyclonal antibody was prepared using as an immunogen the recombinant Nectin-2ED-FLAG protein prepared in EXAMPLE 17. A PBS solution of the Nectin-2ED-FLAG protein was equally mixed with Freund's complete adjuvant. Using the emulsion thus prepared, 0.1 mg/animal of the Nectin-2ED-FLAG protein was immunized into 3 domestic rabbits *(Oryctolagus cuniculus,* female, 3 kg) subcutaneously and intracutaneously at the back of the animal. For the second and subsequent immunization, the protein emulsion was likewise prepared using Freund's incomplete adjuvant and booster was repeated 7 times every 2 weeks.

Prior to the immunization and a week after the fourth and sixth booster, blood was collected through the ear vein. An increase in the antibody titer in sera was confirmed by ELISA using an immunoplate coated with the Nectin-2ED-FLAG protein. A week after the last booster, blood was collected from the 3 rabbits through the carotid artery under anesthesia to give the anti-sera of 78.9 ml, 78.2 ml and 78.8 ml, respectively.

These anti-sera were diluted in PBS to 2-fold and centrifuged. The supernatant was provided for an antigen column prepared by immobilizing the Nectin-2ED-FLAG protein to HiTrap NHS-Activated HP (Amersham Biosciences). After washing with PBS, the column was eluted with 0.1 M Glycine-HCl (pH 3) containing 0.15 M NaCl. The eluate was neutralized with 1 M Tris-HCl (pH 8) and then dialyzed to PBS at 4°C overnight to acquire rabbit anti-Nectin-2 polyclonal antibodies. The purified rabbit anti-Nectin-2 polyclonal antibody samples were named N2-No.1, N2-No.2 and N2-No.3, respectively.

### EXAMPLE 19

### Preparation of rabbit anti-Nectin-2 polyclonal antibody using DNA immunization

Preparation of the polyclonal antibody to the Nectin-2 protein was consigned to Genovac GmbH with expertise in producing antibodies by DNA immunization. For the immunization, cDNA encoding the amino acid sequence of Nectin-2δ (SEQ ID NO: 3) was used. In accordance with the method described in the patent literature (WO 00/29442) filed by Genovac GmbH, 2 rabbits received DNA immunization to give the antisera of 127 mL and 115 mL, respectively.

These antisera were diluted in PBS to 2-fold and centrifuged. The supernatant was provided for an antigen column prepared by immobilizing the Nectin-2ED-FLAG protein to HiTrap NHS-Activated HP (Amersham Biosciences). After washing with PBS, the column was eluted with 0.1 M Glycine-HCl (pH 3) containing 0.15 M NaCl. The eluate was neutralized with 1 M Tris-HCl (pH 8) and then dialyzed to PBS at 4°C overnight to acquire the purified rabbit anti-Nectin-2 polyclonal antibody samples. The rabbit anti-Nectin-2 polyclonal antibodies acquired here were named N2-R1 and N2-R2, respectively.

### EXAMPLE 20

### Detection of Nectin-2 protein in human cancer cell lines

Expression level of the Nectin-2 protein in cancer cells was examined. The following cancer cell lines purchased from ATCC were cultured: NCI-H1703, HT-29, OV-90, SKBR-3, SK-OV-3, NCI-H2342, TOV-112D, NCI-H2122, NCI-H292, Capan-2, MDA-MB-231, BxPC-3, HCT-8, SK-N-DZ, Caov-3, DU 145, A549, Caco-2, WiDr, ZR-75-1, HCT-15, NCI-H1299, NCI-H2228 and BT47. Cell suspensions were thus prepared at 1,000,000 cells/mL, respectively, using Stain buffer (BD Pharmingen). N2-R1 prepared in EXAMPLE 19 was added to the cell suspension in a final concentration of 3 µg/mL and the mixture was allowed to stand at 4°C for an hour. Also, non-immunized rabbit IgG (Jackson) was added to the cell suspension in a final concentration of 3 µg/mL and the mixture was used as negative control. After centrifugation, the system was washed with Stain buffer and Alexa488-labeled anti-rabbit IgG antibody (Molecular Probes) was added in a final concentration of 10 µg/mL. The mixture was allowed to stand at 4°C for an hour. The Nectin-2 protein that appeared in the respective cells was then detected by FACScan (Becton Dickinson). The ratio of the median value of the N2-R1 group to the negative control group is shown in [TABLE 2]. The results indicate that the Nectin-2 protein is detected in the cancer cell lines used.

**[TABLE 2]**

| Cell name | Ratio | Cell name | Ratio |
|---|---|---|---|
| NCI-H1703 | 72.5 | HCT-8 | 48.8 |
| HT-29 | 65.5 | SK-N-DZ | 6.0 |
| OV-90 | 133.4 | Caov-3 | 28.4 |
| SKBR-3 | 61.6 | DU 145 | 17.9 |
| SK-OV-3 | 65.0 | A549 | 16.9 |
| NCI-H2342 | 62.1 | Caco-2 | 50.5 |
| TOV-112D | 44.1 | WDr | 50.0 |
| NCI-H2122 | 28.1 | ZR-75-1 | 25.5 |
| NCI-H292 | 10.6 | HCT-15 | 25.9 |
| Capan-2 | 83.6 | NCI-H1299 | 36.9 |
| MDA-MB-231 | 17.0 | NCI-H2228 | 20.9 |
| BxPC-3 | 46.6 | BT474 | 42.0 |

### EXAMPLE 21

### Establishment of the cell line stably expressing the recombinant full-length Nectin-2δ protein (1)

The cell line constitutively expressing Nectin-2δ protein (SEQ ID NO: 3) was established. A full-length gene for Nectin-2δ was incorporated into glutamine synthetase (GS) expression vector pEE12.4 (Lonza Biologics) to prepare Nectin-2δ expression plasmid (pEE12.4-Nectin-2δ). Linearized pEE12.4-Nectin-2δ was transfected to CHO-K1 cells (10,000,000 cells) using Gene Pulser (Bio-Rad). The cells were resuspended in GS-selective DMEM medium (JRH) containing 10% dialyzed serum (Invitrogen) and GS supplement (JRH), and plated on 40 wells of a 96-well flat-bottomed tissue culture plate at 2500 cells/50 µL/well. After incubation at 37°C for 24 hours in a 5% carbon dioxide gas flow, 150 µL each of the medium above containing 33.3 µM or 66.6 µM MSX (ICN) was added to 20 plates. The incubation was continued at 37°C for 3 to 4 weeks in a 5% carbon dioxide gas flow and the grown colony was plated on a 24-well flat bottomed tissue culture plate. After the incubation was continued, the total RNA was extracted from the 24-well flat bottomed tissue culture plate using RNeasy 96 Kit (QIAGEN), followed by reverse transcription. Quantitative PCR was carried out using the reaction product as a template. Expression level of mRNA for the Nectin-2δ gene was calculated by standardization with the expression level of mRNA for endogenous GAPDH to acquire 60 strains in the order of higher expression level of mRNA for the Nectin-2δ gene.

Expression level of the Nectin-2δ protein in these 60 strains was determined by flow cytometry using AS-2704 prepared in EXAMPLE 16 to acquire CHO cell line 43-2 highly expressing the Nectin-2δ protein.

### EXAMPLE 22

### Establishment of the cell line stably expressing the recombinant full-length

### Nectin-2δ protein (2)

The cell line constitutively expressing Nectin-2δ protein (SEQ ID NO: 3) was established. HT-29 purchased from ATCC was incubated in M5A medium supplemented with 10% fetal bovine serum (JRH). The HT-29 cells were recovered using trypsin-EDTA (Invitrogen). One million of the recovered HT-29 cells were suspended in 100 µl of solution V attached to Cell Line Nucleofector Kit V (Amaxa), which solution contained 2 µg of pcDNA3.1 (+)-Nectin-2δ, and transfected using Nucleofector program T-20. After the incubation was continued for 2 days, the medium was exchanged with the above medium (G418 selection medium) containing 0.5 mg/ml of G418 (Promega). The incubation was continued in the G418 selection medium. After subculturing twice using trypsin/EDTA (Invitrogen), the cells were plated on a 96-well flat-bottomed tissue culture plate at one cell/well and the incubation was continued in the G418 selection medium. The cells were recovered from the wells where colonies were formed and plated on a 24-well flat-bottomed tissue culture plate. After the incubation was continued in the G418 selection medium, the cells were suspended in 200 µL of SDS-PAGE sample buffer (Bio-Rad) containing 1% 2-mercaptoethanol. After heat treatment at 100°C for 3 minutes, 20 µl of the suspension was provided for SDS-PAGE on 7.5% acrylamide gel. Using AS-2706 prepared in EXAMPLE 16, western blotting was performed to give the HT-29 cell line 2δ-25 highly expressing the Nectin-2δ protein.

### EXAMPLE 23

### Study of the cell growth inhibitory activity using rabbit anti-Nectin-2 polyclonal antibody (1)

The CHO cell line 43-2 highly expressing the Nectin-2δ, which was prepared in EXAMPLE 21, was suspended in GS-selective DMEM medium (JRH) containing 10% dialyzed serum (Invitrogen), GS supplement (JRH) and 25 µM MSX (ICN), and plated on a 96-well flat-bottomed tissue culture plate at 250 cells/well or 500 cells/well. After incubation at 37°C for 24 hours in a 5% carbon dioxide gas flow, the rabbit anti-Nectin-2 polyclonal antibody N2-No.1, N2-No.2, N2-No.3, N2-R1 or N2-R2 obtained in EXAMPLE 18 and EXAMPLE 19 was added in a final concentration of 30 µg/mL. For negative control, non-immunized rabbit IgG (Jackson) was added in the same concentration. The incubation was carried out at 37°C for 3 days in a 5% carbon dioxide gas flow, and effects of the rabbit anti-Nectin-2 polyclonal antibody on the cell growth were observed using WST-8 (Dojindo). When the cells were plated in 250 cells/well, decrease in absorbance value was detected in the groups added with N2-No.1, N2-No.2, N2-No.3, N2-R1 or N2-R2, which decrease was 81%, 76%, 78%, 80% and 85%, respectively, as compared to the negative control group. These results indicate that the anti-Nectin-2 polyclonal antibodies had the cell growth inhibitory activity on the CHO cell line 43-2 highly expressing the Nectin-2δ protein.

### EXAMPLE 24

### Study of the cell growth inhibitory activity using rabbit anti-Nectin-2 polyclonal antibody (2)

The HT-29 cell line 2δ-25 highly expressing the Nectin-2δ, which was prepared in EXAMPLE 22, was suspended in M5A medium supplemented with 1% fetal bovine serum (JRH), and plated on a 96-well flat bottom tissue culture plate at 1,000 cells/well. At the same time when the cells were plated, the rabbit anti-Nectin-2 polyclonal antibody N2-No.1 obtained in EXAMPLE 19 was added in a final concentration of 30 µg/mL. For negative control, non-immunized rabbit IgG (Jackson) was added in the same concentration. Incubation was performed at 37°C for 6 days in a 5% carbon dioxide gas flow, and effects of the anti-Nectin-2 polyclonal antibody on the cell growth were observed using WST-8 (Dojindo). In the group added with the rabbit anti-Nectin-2 polyclonal antibody N2-No.1, a 36% decrease in absorbance value was detected, as compared to the negative control group. The results indicate that the anti-Nectin-2 polyclonal antibody had the cell growth inhibitory activity on the HT-29 cell line 2δ-25 highly expressing the Nectin-2δ protein.

### INDUSTRIAL APPLICABILITY

The protein comprising the same or substantially the same as the amino acid sequence represented by SEQ ID NO: 1, SEQ ID NO: 3, SEQ ID NO: 17, SEQ ID NO: 25, SEQ ID NO: 38 or SEQ ID NO: 48 (the protein used in the present invention) or the polynucleotide encoding this protein is expressed specifically in cancer tissues and acts as a diagnostic marker for cancer. Thus, the antibody to said protein, the antisense polynucleotide for said polynucleotide, the double stranded RNA comprising a part of RNA encoding said protein, the compound or its salt that inhibits the activity of said protein, the compound or its salt that inhibits the expression of a gene for said protein, etc. can be safely used as an agent for preventing/treating a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably an agent for preventing/treating breast cancer, lung cancer, colon cancer, prostate cancer, ovary cancer, pancreatic cancer, etc.), an apoptosis promoter in cancer cells, a growth inhibitor in cancer cells, an inducer of cell cycle change in cancer cells, and so on. Also, the protein, polynucleotide and antibody described above and the like are useful for screening an agent for preventing/treating a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably an agent for preventing/treating breast cancer, lung cancer, colon cancer, prostate cancer, ovary cancer, pancreatic cancer, etc.), an apoptosis promoter in cancer cells, a growth inhibitor in cancer cells, an inducer of cell cycle change in cancer cells, and so on.

## Claims

1. An agent for preventing/treating cancer, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

2. An apoptosis promoter of cancer cells, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

3. A growth inhibitor of cancer cells, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

4. A diagnostic product for cancer, which comprises an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

5. An agent for preventing/treating cancer, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

6. An apoptosis promoter of cancer cells, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

7. A growth inhibitor of cancer cells, which comprises (i) an antisense polynucleotide comprising a base sequence or a part thereof, complementary or substantially complementary to the base sequence of a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or (ii) a double-stranded RNA comprising a part of RNA encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

8. An agent for preventing/treating cancer, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

9. An agent for preventing/treating cancer, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

10. An agent for preventing/treating cancer, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

11. An apoptosis promoter of cancer cells, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

12. An apoptosis promoter of cancer cells, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

13. An apoptosis promoter of cancer cells, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

14. A growth inhibitor of cancer cells, which comprises a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

15. A growth inhibitor of cancer cells, which comprises a compound or its salt that inhibits the expression of a gene for a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

16. A growth inhibitor of cancer cells, which comprises a compound or its salt that inhibits the expression of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48.

17. A diagnostic product for cancer, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

18. A method of screening an agent for preventing/treating cancer, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

19. A method of screening an agent for preventing/treating cancer, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 3 8 or SEQ ID NO: 48, or its partial peptide.

20. A method of screening an apoptosis promoter of cancer cells, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

21. A method of screening an apoptosis promoter of cancer cells, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

22. A method of screening a growth inhibitor of cancer cells, which comprises using a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

23. A method of screening a growth inhibitor of cancer cells, which comprises using a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

24. A kit for screening an agent for preventing/treating cancer, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

25. A kit for screening an agent for preventing/treating cancer, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

26. A kit for screening an apoptosis promoter of cancer cells, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

27. A kit for screening an apoptosis promoter of cancer cells, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

28. A kit for screening a growth inhibitor of cancer cells, which comprises a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

29. A kit for screening a growth inhibitor of cancer cells, which comprises a polynucleotide encoding a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide.

30. A method for preventing/treating cancer, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

31. A method for promoting apoptosis of cancer cells, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

32. A method for inhibiting growth of cancer cells, which comprises administering to a mammal an effective dose of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

33. A method for preventing/treating cancer, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

34. A method for promoting apoptosis of cancer cells, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

35. A method for inhibiting growth of cancer cells, which comprises inhibiting the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof.

36. Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an agent for preventing/treating cancer.

37. Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an apoptosis promoter of cancer cells.

38. Use of an antibody to a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture a growth inhibitor of cancer cells.

39. Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, or its partial peptide, or a salt thereof, to manufacture an agent for preventing/treating cancer.

40. Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture an apoptosis promoter of cancer cells.

41. Use of a compound or its salt that inhibits the activity of a protein comprising the same or substantially the same amino acid sequence as the amino acid sequence represented by SEQ ID NO: 1, SEQ ID NO: 3, SEQ ID NO: 17, SEQ ID NO: 25, SEQ ID NO: 38 or SEQ ID NO: 48, its partial peptide, or a salt thereof, to manufacture a growth inhibitor of cancer cells.
